# EUROPÄISCHE PATENTSCHRIFT

(11) **EP 4 237 032 B1**
(45) Veröffentlichungstag und Bekanntmachung des Hinweises auf die Patenterteilung: **20.05.2026**
(21) Anmeldenummer: 21801128.6
(22) Anmeldetag: 27.10.2021
(51) Int. Cl.: A61M 1/16, A61M 39/22

(54) **VENTILVORRICHTUNG, EFFLUENTBEUTEL UND VERFAHREN**
VALVE DEVICE, EFFLUENT BAG AND METHODS
ENSEMBLE CLAPET, POCHE À EFFLUENTS ET PROCÉDÉ

(30) Priorität: 28.10.2020 DE 102020128296
(43) Veröffentlichungstag der Anmeldung: 06.09.2023
(73) Patentinhaber: Fresenius Medical Care Deutschland GmbH, 61352 Bad Homburg (DE)
(72) Erfinder: KLEWINGHAUS, Juergen, 61440 Oberursel (DE)
(74) Vertreter: Bobbert & Partner Patentanwälte PartmbB
(86) Internationale Anmeldenummer: PCT/EP2021/079863
(87) Internationale Veröffentlichungsnummer: WO 2022/090332

(56) Entgegenhaltungen:
- DE-A1- 102017 116 142
- TADANO KOTARO ET AL: "Development of a Four-Way Pinch-Type Servo Valve for Pneumatic Actuator", APPLIED SCIENCES, vol. 10, no. 3, 5 February 2020 (2020-02-05), pages 1066, XP055876589, ISSN: 2076-3417, Retrieved from the Internet <URL:http://dx.doi.org/10.3390/app10031066> [retrieved on 20220106], DOI: 10.3390/app10031066

## Beschreibung

Die vorliegende Erfindung betrifft eine Ventilvorrichtung gemäß Anspruch 1 und einen Effluentbeutel gemäß Anspruch 11. Sie betrifft zudem ein Set gemäß Anspruch 12 sowie ein Verfahren gemäß Anspruch 14 zum Vorbereiten eines Effluentbeutels und ein Verfahren gemäß Anspruch 15 zum Entleeren des Effluentbeutels, ferner eine Blutbehandlungsvorrichtung gemäß Anspruch 16 bzw. gemäß jeweils der Oberbegriffe oder Gattungsbegriffe dieser Ansprüche.

Aus der Praxis ist die extrakorporale Blutbehandlung bekannt. Dabei wird dem Patienten Blut entnommen, das entlang eines Blutkreislaufs extrakorporal und z. B. durch einen Blutfilter geführt wird. Der Blutfilter weist eine Blutkammer, durch welche Blut geführt wird, und eine Dialysierflüssigkeitskammer, durch welche Dialysierflüssigkeit geführt wird, auf. Beide Kammern sind durch eine semi-permeable Membran voneinander getrennt. Blut und Dialysierflüssigkeit werden zumeist im Gegenstromprinzip durch den Blutfilter geleitet. Das Blut wird im Blutfilter gereinigt, die Dialysierflüssigkeit gilt bei ihrem Austritt aus dem Blutfilter als verbraucht und wird, nun als Dialysat bezeichnet, verworfen. Neben dem Dialysat umfasst das zu verwerfende Fluid auch Filtrat, welches Wasser, das dem Blut im Blutfilter entzogen wurde, umfasst. Filtrat und Dialysat werden im Folgenden einzeln oder gemeinsam vereinfacht als Effluent bezeichnet.

Das Effluent wird in der Praxis mittels einer Effluentzulaufleitung einem Effluentbeutel zugeführt und darin zunächst aufbewahrt. Nach Beendigung der Blutbehandlung, oder in Beutelleerintervallen (Intervalle, in welchen der Beutel geleert wird) während der Blutbehandlung, wird das Effluent aus dem Effluentbeutel, der über ein Waschbecken oder einen Ausguss gehalten wird, in diesen hinein verworfen. DE 10 2017 116142 A1 offenbart ein Effluentbeutel-System mit einem Drei-Wege-Hahn als Umschalteinrichtung zur Steuerung des Effluentflusses zwischen Befüllen und Entleeren, wobei der Hahn einen Griffabschnitt für manuelle Betätigung aufweist und zwischen verschiedenen Stellungen schaltbar ist.

TADANO KOTARO ET AL: "Development of a Four-Way Pinch-Type Servo Valve for Pneumatic Actuator",APPLIED SCIENCES,Bd. 10, Nr. 3 5. Februar 2020 (2020-02-05), Seite 1066, XP055876589,ISSN: 2076-3417, DOI: 10.3390/app10031066 offenbart ein Pinch-Typ Ventil mit NockenMechanismus zum Blockieren/Freigeben von Fluidleitungen durch mechanisches Zusammendrücken elastischer Schläuche, wobei Stellelemente zwischen verschiedenen Positionen schaltbar sind zur Flusssteuerung in Pneumatiksystemen.

Eine Aufgabe der vorliegenden Erfindung ist es, eine Ventilvorrichtung zur Verwendung mit einem bei der Blutbehandlung eingesetzten Effluentbeutel und einen weiteren Effluentbeutel zum Einsatz bei der Blutbehandlung anzugeben.

Ferner sollen ein Set mit einer erfindungsgemäßen Ventilvorrichtung, ein Verfahren zum Vorbereiten des Effluentbeutels für eine anstehende Blutbehandlung, ein Verfahren zum Entleeren des Effluentbeutels und eine Blutbehandlungsvorrichtung angegeben werden.

Die erfindungsgemäße Aufgabe wird durch eine Ventilvorrichtung mit den Merkmalen des Anspruchs 1 und mit einem Effluentbeutel mit den Merkmalen des Anspruchs 11 gelöst. Zudem wird sie gelöst durch ein Set mit den Merkmalen des Anspruchs 12 sowie durch ein Verfahren zum Vorbereiten eines Effluentbeutels mit den Merkmalen des Anspruchs 14 und ein Verfahren zum Entleeren des Effluentbeutels mit den Merkmalen des Anspruchs 15, ferner durch eine Blutbehandlungsvorrichtung mit den Merkmalen des Anspruchs 16.

Die vorliegende Erfindung betrifft eine Ventilvorrichtung, ausgestaltet und vorgesehen zu ihrem Verbinden mit einem Auslasshahn, welcher einer Effluentauslassöffnung eines Effluentbeutels zum Aufnehmen von bei einer Blutbehandlung anfallenden Effluents zugeordnet oder hiermit verbundenen ist. Ein solcher Auslasshahn, mit welchem die Ventilvorrichtung für ihren Gebrauch bestimmungsgemäß verbunden werden kann, ist zumeist bereits in oder an einer mit der Effluentauslassöffnung verbundenen Effluentablaufleitung, über welche im Gebrauch das im Effluentbeutel vorhandene Effluent ganz oder teilweise ablaufen kann, um verworfen zu werden, oder einem Ansatz für eine solche Effluentablaufleitung angeordnet. Ein solcher Auslasshahn weist ein Stellelement auf, welches in eine erste Stellung, in welcher der Fluss aus der Effluentauslassöffnung entlang der Effluentablaufleitung gesperrt ist, und in eine zweite Stellung, in welcher der Fluss aus der Effluentauslassöffnung entlang der Effluentablaufleitung und aus dem Effluentbeutel heraus freizugeben ist, gebracht werden kann.

Die Ventilvorrichtung weist einen Halteabschnitt (alternativ: Verbindungsabschnitt) auf, mittels welchem die Ventilvorrichtung, vorzugsweise form- und/oder kraftschlüssig, an oder auf dem Auslasshahn gehalten wird oder gehalten oder mit diesem verbunden werden kann.

Ferner weist die Ventilvorrichtung einen Einlegeabschnitt auf, welcher zum Einlegen einer zweiten Fluidleitung oder eines Abschnitts hiervon in die Ventilvorrichtung oder eines Abschnitts hiervon dient. Die zweite Fluidleitung kann hierin auch als Verbindungsleitung oder "Verlängerung" einer nachstehend detaillierter diskutierten Effluentzulaufleitung, über welche Effluent von der Dialysierflüssigkeitskammer der Behandlungsvorrichtung kommend in den Effluentbeutel zugeführt oder zugeleitet wird, bezeichnet werden.

Weiter weist die Ventilvorrichtung ein Sperrelement auf, welches zwischen wenigstens einer ersten und einer zweiten Position schaltbar ist. Hierbei ist das Sperrelement angeordnet, um in der ersten Position direkt oder indirekt auf die zweite Fluidleitung einzuwirken, um einen Fluss entlang der zweiten Fluidleitung zu unterbinden, und um, im Gebrauch, vorzugsweise eine elektrisch leitfähige Flüssigkeitssäule in der zweiten Fluidleitung elektrisch isolierend zu unterbrechen und/oder um die Fluidleitung zu sperren, und um in der zweiten Position einen Fluss entlang der zweiten Fluidleitung zuzulassen.

Die vorliegende Erfindung betrifft ferner einen Effluentbeutel, welcher ausgestaltet ist, um während einer Blutbehandlung anfallendes Effluent aufzunehmen und welcher außerdem eine erfindungsgemäße Ventilvorrichtung aufweist.

Der erfindungsgemäße Effluentbeutel weist hierbei eine, vorzugsweise verschließbare, Effluenteinlassöffnung und eine, vorzugsweise verschließbare, hiervon getrennte Effluentauslassöffnung auf. Effluenteinlassöffnung und Effluentauslassöffnung dienen der Verbindung von einem Inneren des Effluentbeutels zu einem Äußeren des Effluentbeutels zum Zu- bzw. Abführen von Effluent in bzw. aus dem Effluentbeutel.

Weiter umfasst der erfindungsgemäße Effluentbeutel den mit der Effluentauslassöffnung verbundenen oder noch zu verbindenden Auslasshahn, welcher ein Stellelement aufweist und in oder an einer Effluentablaufleitung angeordnet ist, wobei das Stellelement in eine erste Stellung des Auslasshahns, in welcher der Fluss aus der Effluentauslassöffnung entlang der Effluentablaufleitung gesperrt ist, und in eine zweite Stellung, in welcher der Fluss aus der Effluentauslassöffnung entlang der Effluentablaufleitung und aus dem Effluentbeutel heraus freizugeben ist, gebracht werden kann.

Ein erfindungsgemäßes Set umfasst eine erfindungsgemäße Ventilvorrichtung und eine zweite Fluidleitung zum Einlegen in den Einlegeabschnitt der erfindungsgemäßen Ventilvorrichtung.

Das erfindungsgemäße Verfahren dient zum Vorbereiten eines Effluentbeutels zum Aufnehmen von bei einer Blutbehandlung anfallenden Effluents.

Dieses Verfahren umfasst das Bereitstellen eines erfindungsgemäßen Effluentbeutels oder eines erfindungsgemäßen Sets.

Weiter umfasst das Verfahren das Verbinden der zweiten Fluidleitung sowohl mit der Effluentzulaufleitung als auch mit der Effluenteinlassöffnung. Geeignete Verbinder sind hierzu an den freien Enden der zweiten Fluidleitung des Effluentbeutels oder der Ventilvorrichtung des Sets vorgesehen.

Das Einlegen der zweiten Fluidleitung in den Einlegeabschnitt der Ventilvorrichtung, sofern noch nicht geschehen, ist ebenfalls von der vorliegenden Erfindung umfasst.

Schließlich umfasst die vorliegende Erfindung das Verbinden der Ventilvorrichtung mit dem Auslasshahn mittels des Halteabschnitts oder Verbindungsabschnitts der Ventilvorrichtung.

Das erfindungsgemäße Verfahren zum Entleeren eines Effluentbeutels umfasst das Bereitstellen eines erfindungsgemäßen Effluentbeutels oder eines erfindungsgemäßen Sets, vorzugsweise jeweils vorbereitet gemäß dem vorstehend genannten, erfindungsgemäßen Verfahren zum Vorbereiten des Effluentbeutels.

Ferner umfasst das zweite Verfahren das Betätigen eines Umschaltelements der erfindungsgemäßen Ventilvorrichtung derart, dass eine Fluidverbindung zwischen dem Inneren des Effluentbeutels und dem Inneren der Effluentablaufleitung hergestellt wird.

Alle mit den erfindungsgemäßen Verfahren erzielbaren Vorteile lassen sich in bestimmten erfindungsgemäßen Ausführungsformen ungeschmälert auch mit den erfindungsgemäßen Vorrichtungen erzielen, und umgekehrt.

Die erfindungsgemäße Blutbehandlungsvorrichtung ist mit einem erfindungsgemäßen Effluentbeutel oder mit einem erfindungsgemäßen Set verbunden, wobei der erfindungsgemäße Effluentbeutel oder der Effluentbeutel des erfindungsgemäßen Sets vorzugsweise jeweils vorbereitet ist gemäß dem erfindungsgemäßen Verfahren zum Vorbereiten des Effluentbeutels.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche und Ausführungsformen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt diese eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, **z. B.** im Sinne eines Disclaimers. Für jede hierin genannte Ausführungsform gilt somit, dass die gegenteilige Ausführungsform, beispielsweise als Negation formuliert, ebenfalls offenbart ist.

Erfindungsgemäße Ausführungsformen können eines, mehrere oder alle der oben und/oder im Folgenden genannten Merkmale in jeder technisch möglichen Kombination aufweisen.

In manchen Ausführungsformen weist der Auslasshahn eine Durchgangsöffnung oder ein Durchgangslumen auf zum Hindurchführen eines Abschnitts einer Effluentablaufleitung oder eines in der Effluentablaufleitung strömenden Effluents durch den Auslasshahn. Der Auslasshahn kann einen Griffabschnitt aufweisen, welcher im bestimmungsgemäßen Gebrauch dazu dient, mit seiner Hilfe ein Stellelement des Auslasshahns manuell von dessen ersten Stellung in dessen zweite Stellung überzuführen, und umgekehrt.

In einigen Ausführungsformen der erfindungsgemäßen Ventilvorrichtung stehen Ventilvorrichtung und/oder Auslasshahn nicht in Wirk- oder Fluidverbindung mit einer pneumatischen Antriebsvorrichtung.

In manchen Ausführungsformen der erfindungsgemäßen Ventilvorrichtung ist ihr Sperrelement angeordnet, um dann, wenn das Stellelement des Auslasshahns in die erste Stellung übergeführt wird, zwangsgeführt in die zweite Position übergeführt zu werden, und um dann, wenn das Stellelement in die zweite Stellung übergeführt wird, zwangsgeführt in die erste Position übergeführt zu werden.

In einigen Ausführungsformen ist das Stellelement des Auslasshahns angeordnet, um dann, wenn das Sperrelement in die erste Position übergeführt wird, zwangsgeführt in die zweite Stellung übergeführt zu werden, und um dann, wenn das Sperrelement in die zweite Position übergeführt wird, zwangsgeführt in die erste Stellung übergeführt zu werden.

In manchen Ausführungsformen ist das Sperrelement angeordnet, um dann, wenn das Stellelement des Auslasshahns in die zweite Stellung übergeführt wird, zwangsgeführt bereits eine Position einzunehmen oder diese beizubehalten, in welcher ein Fluss entlang der zweiten Fluidleitung unterbunden ist oder bleibt und im Gebrauch vorzugsweise eine elektrisch leitfähige Flüssigkeitssäule in der zweiten Fluidleitung elektrisch isolierend unterbrochen ist bzw. die Fluidleitung gesperrt ist oder bleibt, bevor das Stellelement die zweite Stellung einnimmt oder erstmals einen Fluss durch die Effluentablaufleitung erlaubt.

In einigen Ausführungsformen weist der Halteabschnitt der erfindungsgemäßen Ventilvorrichtung einen Halter, einen Drehriegel und einen Deckel auf. Hierbei weist der Halter den Einlegeabschnitt für die zweite Fluidleitung auf, welcher mit dem Drehriegel, welcher als Sicherung gegen ein Entnehmen der zweiten Fluidleitung aus der Ventilvorrichtung dient, verschlossen werden kann.

Hierbei kann der Deckel am Halter vorgesehen sein, und zwar derart, dass der Deckel zwischen einer ersten, geöffneten Deckelstellung zum Einlegen oder Herausnehmen des Auslasshahns und ggf. auch eines Abschnitts der ersten Fluidleitung in den oder aus dem Halteabschnitt oder dem Halter, und einer zweiten, geschlossenen Deckelstellung zum kraft- und/oder formschlüssigen Halten des Auslasshahns im Halteabschnitt, bewegbar ist.

In manchen Ausführungsformen weist der Halteabschnitt einen Halter (z. B. den vorstehend genannten) zur Aufnahme des Auslasshahns oder eines Abschnitts hiervon und einen Deckel (z. B. den vorstehend genannten) auf, wobei Halter und/oder Deckel mit einem Schnapp- oder Klicksystem ausgestaltet sind. Das Schnapp- oder Klicksystem dient zu ihrem gegenseitigen bzw. gemeinsamen Verbinden und/oder zum zumindest teilweisen Verschließen des Halteabschnitts bzw. des Halters mittels des Deckels.

In einigen Ausführungsformen der erfindungsgemäßen Ventilvorrichtung ist die zweite Fluidleitung oder ein Abschnitt hiervon in den Einlegeabschnitt eingelegt.

In manchen Ausführungsformen ist der Auslasshahn oder ein Abschnitt hiervon in den Halteabschnitt der Ventilvorrichtung eingelegt. Alternativ ist der Halteabschnitt mit dem Auslasshahn verbunden.

In einigen Ausführungsformen weist die zweite Fluidleitung ein erstes freies Ende zu ihrem Verbinden mit einer Effluentzulaufleitung und ein zweites freies Ende zu ihrem Verbinden mit einer Effluenteinlassöffnung eines Effluentbeutels (oder einer hiermit verbundenen Fluidleitung) auf. Die Enden können Verbinder z. B. Luer-Lockverbinder oder von einem anderen Typ sein.

In manchen Ausführungsformen weist die erfindungsgemäße Ventilvorrichtung weiter ein Umschaltelement auf. Das Umschaltelement ist vorgesehen und angeordnet zum manuellen - direkten oder indirekten - Umschalten des Sperrelements von der ersten Position in die zweite Position und umgekehrt.

Hierbei ist das Umschaltelement vorzugsweise derart angeordnet, dass bei manuellem Umschalten des Sperrelements von der ersten Position in die zweite Position, das Stellelement oder ein Verbindungsabschnitt zum Verbinden des Umschaltelements mit dem Stellelement von der zweiten Stellung in die erste Stellung übergeführt wird und umgekehrt.

In einigen Ausführungsformen ist das Sperrelement angeordnet, um bei seiner Rotation um einen ersten Winkel α1 zwischen einer zweiten Position, in welcher die zweite Fluidleitung maximal vom Sperrelement freigegeben ist, und einer ersten Position, in welcher die zweite Fluidleitung maximal durch das Sperrelement geschlossen ist, übergeführt zu werden. Das Stellelement kann dabei angeordnet sein, um bei seiner Rotation um einen zweiten Winkel α2 zwischen einer ersten Stellung, in welcher die Effluentablaufleitung maximal vom Stellelement verschlossen ist, in eine zweite Stellung, in welcher das Stellelement erstmals Fluss durch die Effluentablaufleitung erlaubt und hierdurch geöffnet wird, übergeführt zu werden. Der erste Winkel α1 ist dabei kleiner als der zweite Winkel α2.

In manchen Ausführungsformen steht die Effluentablaufleitung in Förderverbindung mit wenigstens einer Pumpe und/oder einem Pumpenantrieb einer Pumpe.

In einigen Ausführungsformen des erfindungsgemäßen Sets, weist dieses weiter einen Effluentbeutel zum Aufnehmen von bei einer Blutbehandlung anfallenden Effluents auf. Der Effluentbeutel weist seinerseits eine Effluenteinlassöffnung und eine hiervon getrennte Effluentauslassöffnung sowie einen Auslasshahn zum Verschließen der Effluentauslassöffnung, oder einen Ansatz für den Auslasshahn, auf. Die Ventilvorrichtung ist vorzugsweise konfiguriert, um mittels ihres Halteabschnitts den Auslasshahn, vorzugsweise form- und/oder kraftschlüssig, zu halten.

In manchen Ausführungsformen weist die Pumpe oder der Pumpenantrieb in der Effluentablaufleitung wenigstens einen magnetisch gelagerten oder angetriebenen Pumpabschnitt, insbesondere einen Pumpkopf, auf. Dieser Pumpabschnitt oder Pumpkopf ist beispielsweise ausgestaltet als Impellerpumpkopf oder als dessen Rotor.

In manchen Ausführungsformen ist die erfindungsgemäße Blutbehandlungsvorrichtung als Hämodialysevorrichtung, Hämofiltrationsvorrichtung oder Hämodiafiltrationsvorrichtung, insbesondere als eine Vorrichtung für die Anwendung für die kontinuierliche venöse Hämodiafiltration (CVV-HDF) und/oder zur Anwendung für die Akutdialyse ausgestaltet.

In einigen Ausführungsformen umfasst die Blutbehandlungsvorrichtung optional eine Ladestation für eine Spannungsquelle für den Pumpenantrieb der Pumpe. Die Spannungsquelle kann eine Niedervolt- oder Niederstrom-Quelle sein.

In einigen Ausführungsformen kann der Effluentbeutel ein Behälter jeder Art sein, beispielsweise ein Container mit flexibler Außenhaut wie einer Folie, oder aus Folie, ein Container mit einer harten Außenhaut, oder aus einer harten Außenhaut, wie ein Kanister, usw.

In gewissen Ausführungsformen wird der Auslasshahn derart betätigt, dass eine Fluidverbindung, welche zwischen dem Inneren des Effluentbeutels und dem Inneren der Effluentablaufleitung besteht, unterbrochen wird.

In manchen Ausführungsformen wirkt der Auslasshahn elektrisch isolierend. Hierunter kann vorzugsweise verstanden werden, dass Fluide, die mittels eines Verbinders oder Anschlusses des Auslasshahns in diesen eindringen können (wie etwa der Effluentzustrom in den Effluentbeutel hinein), und jene Flächen, welche sie dabei berühren, mit anderen Fluiden, die mittels eines anderen Verbinders oder Anschlusses des Auslasshahns in letztere eindringen können (wie etwa der Effluentabstrom aus dem Effluentbeutel heraus), und jene Flächen, welche sie dabei berühren, für die Übertragung von elektrischem Strom nicht miteinander in Kontakt kommen können und/oder Strom nicht übertragen.

In einigen Ausführungsformen kann die zweite Fluidleitung werksseitig eingelegt sein, d. h. die zweite Fluidleitung ist bei Lieferung bereits eingelegt und gegen Entnahme gesichert, beispielsweise durch Verbinden oder Verschnappen zweier Komponenten, insbesondere Gehäuseteile, z. B. des Drehriegels und des Halters, miteinander, wobei das Verbinden oder Verschnappen ein nachfolgendes Entnehmen der zweiten Fluidleitung aus der Ventilvorrichtung verhindert.

In manchen Ausführungsformen kann die zweite Fluidleitung nicht aus der Ventilvorrichtung entfernt werden, jedenfalls nicht zerstörungsfrei, beispielsweise durch eine Gestaltung mit Hinterschnitt.

In bestimmten Ausführungsformen kann vorgesehen sein, die Betätigung des Umstellelements ausschließlich zuzulassen, wenn in den Einlegeabschnitt der Ventilvorrichtung tatsächlich eine zweite Fluidleitung eingelegt ist. Dies kann beispielweise mittels eines Pins, auf welchen die zweite Fluidleitung drückt, realisiert werden. Wird der Pin nicht durch die zweite Fluidleitung gedrückt, dann kann der Umschalthebel vorzugsweise nicht betätigt werden.

Manche oder alle erfindungsgemäßen Ausführungsformen können einen, mehrere oder alle der oben und/oder im Folgenden genannten Vorteile aufweisen.

Erfindungsgemäß wird vorgeschlagen, dass zu jedem Zeitpunkt entweder die Effluentzulaufleitung oder die Effluentablaufleitung isolierend unterbrochen ist. Dadurch wird vorteilhaft zuverlässig sichergestellt, dass zu keinem Zeitpunkt eine leitfähige Verbindung zwischen der Effluentzulaufleitung und dem Abfluss besteht, d. h. gesichert zu keinem Zeitpunkt eine Erdung des Patienten gegen den Abfluss erfolgt. Damit wird vorteilhafterweise das Risiko umgangen, dass beim Ablassen des Inhalts des Effluentbeutels ein elektrisch leitender Kontakt von der Flüssigkeit zur Erde auftritt, sodass die zulässigen Patientenableitströme überschritten würden. Dies trägt vorteilhaft zur Erhöhung der Patientensicherheit bei.

Ein Vorteil der vorliegenden Erfindung kann darin bestehen, dass bei dem erfindungsgemäßen Effluentbeutel, anders als es bei herkömmlichen Effluentbeuteln der Fall ist, nicht aus Sicherheitsgründen die Effluentzulaufleitung manuell vom Effluentbeutel getrennt werden muss, um eine fluidische Trennung des Beutels bzw. seines Inhalts vom Patienten sicherzustellen, wenn der Effluentbeutel bei seinem Entleeren an der Wiegeeinrichtung der Behandlungsvorrichtung verbleiben soll. Gleichwohl kann mittels der vorliegenden Erfindung sichergestellt werden, dass auch in dem Fall, dass der Effluentbeutel an der Behandlungsvorrichtung und mit dieser verbunden bleibt, eine Gefährdung des Patienten durch elektrische Ströme ausgeschlossen werden kann.

Ein weiterer Vorteil der vorliegenden Erfindung besteht im geringen Aufwand, der zur Implementierung der Erfindung erforderlich ist.

Ein mittels der Erfindung erzielter Vorteil besteht darin, dass beide Aktionen, nämlich sowohl das Befüllen als auch das Entleeren des Effluentbeutels, durch Integration der hierfür zu betätigenden Bauteile in einem einzigen Bauteil miteinander gekoppelt und somit für eine gemeinsame Betätigung miteinander verbunden sind. Die Zwangskopplung trägt dazu bei, manuelle Schritte im Zusammenhang mit dem Entleeren nicht vergessen zu können. Zudem spart man sich manuelle oder automatische Schritte ein. Die Flusswege zwischen Ausguss oder Abfluss einerseits und Dialysator oder Patient andererseits können in der vorliegenden Erfindung vorteilhafterweise elektrisch voneinander getrennt sein, was der Sicherheit des Patienten dient.

Ferner ist es von Vorteil, dass die Ventilvorrichtung vorgesehen ist, um manuell betätigt zu werden. Daher erfordert die vorliegende Erfindung keinen Eingriff in die Steuerung oder Regelung der Blutbehandlungsvorrichtung und erlaubt auch deshalb vorteilhaft eine kostengünstige Nachrüstung bestehender Systeme.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt in vereinfachter Darstellung eine erfindungsgemäße Blutbehandlungsvorrichtung mit einem extrakorporalen Blutkreislauf in einer exemplarischen Ausführungsform;
- **Fig. 2a**: zeigt in vereinfachter Darstellung eine Vielzahl von Leitungen und die Funktionsweise eines erfindungsgemäßen Sets mit einer angedeuteten erfindungsgemäßen Ventilvorrichtung und einem erfindungsgemäßen Effluentbeutel in einem Zustand, in welchem dem Effluentbeutel mittels der Effluentzulaufleitung Effluent zugeführt werden kann;
- **Fig. 2b**: zeigt in vereinfachter Darstellung die Leitungen und das erfindungsgemäße Set der Fig. 2a in einem Zustand, in welchem der Effluentbeutel mittels der Effluentablaufleitung entleert werden kann;
- **Fig. 3**: zeigt in schematisch vereinfachter Darstellung einen erfindungsgemäßen Effluentbeutel in einer ersten Ausführungsform mit einer erfindungsgemäßen Ventilvorrichtung, welche in einer zweiten Ausführungsform gezeigt ist;
- **Fig. 4**: zeigt in einer Draufsicht die erfindungsgemäße Ventilvorrichtung der Fig. 3 in Vergrößerung;
- **Fig. 5a**: zeigt in schematisch vereinfachter Darstellung eine dritte Ausführungsform der erfindungsgemäßen Ventilvorrichtung teils in einem Querschnitt zusammen mit einem Auslasshahn in einem unmontierten Zustand, in welchem der Auslasshahn nicht in die Ventilvorrichtung eingelegt ist;
- **Fig. 5b**: zeigt in schematisch vereinfachter Darstellung die erfindungsgemäße Ventilvorrichtung der Fig. 5a teils in einem Querschnitt zusammen mit dem Auslasshahn im montierten Zustand, in welchem der Auslasshahn in die Ventilvorrichtung eingelegt ist;
- **Fig. 6a**: zeigt die Querschnittdarstellung der erfindungsgemäßen Ventilvorrichtung der Fig. 5a oder 5b im montierten Zustand zusammen mit einem Auslasshahn in einem ersten Zustand;
- **Fig. 6b**: zeigt in Schnittdarstellung die Ventilvorrichtung der Fig. 6a im montierten Zustand zusammen mit einem Auslasshahn in einem zweiten Zustand;
- **Fig. 7a**: zeigt eine Ansicht von oben auf ein Sperrelement einer exemplarischen erfindungsgemäßen Ventilvorrichtung in einer ersten Position, in welcher eine durch die Ventilvorrichtung verlaufende zweite Fluidleitung geöffnet ist;
- **Fig. 7b**: zeigt eine Ansicht von oben auf das Sperrelement der Fig. 7a in einer zweiten Position, in welcher die zweite Fluidleitung geschlossen ist;
- **Fig. 8a**: zeigt eine Ansicht von oben auf einen Auslasshahn, links von außen, rechts in einer angedeuteten Längsschnittdarstellung, in welcher die Effluentablaufleitung geschlossen ist;
- **Fig. 8b**: zeigt eine Ansicht von oben auf einen Auslasshahn, rechts von außen, links in einer angedeuteten Längsschnittdarstellung, in der die Effluentablaufleitung geöffnet ist;
- **Fig. 9a**: zeigt einen Längsschnitt mit Ansicht von oben auf ein Sperrelement der erfindungsgemäßen Ventilvorrichtung in einer weiteren Ausführungsform;
- **Fig. 9b**: zeigt einen Längsschnitt mit Ansicht von oben auf ein Stellelement eines Auslasshahns;
- **Fig. 10a**: zeigt einen Querschnitt durch eine erste Ausführungsform eines Sperrelements der erfindungsgemäßen Ventilvorrichtung in dessen zweiter Position;
- **Fig. 10b**: zeigt das Sperrelement aus Fig. 10a in dessen erster Position;
- **Fig. 11a**: zeigt einen Querschnitt durch eine zweite Ausführungsform eines Sperrelements der erfindungsgemäßen Ventilvorrichtung in dessen zweiter Position;
- **Fig. 11b**: zeigt das Sperrelement aus Fig. 11a in dessen erster Position;
- **Fig. 12a**: zeigt einen beispielhaften Verlauf eines erfindungsgemäßen Verfahrens zum Vorbereiten eines Effluentbeutels zum Aufnehmen von bei einer Blutbehandlung anfallenden Effluents; und
- **Fig. 12b**: zeigt einen beispielhaften Verlauf eines Verfahrens zum Entleeren eines erfindungsgemäßen Effluentbeutels.

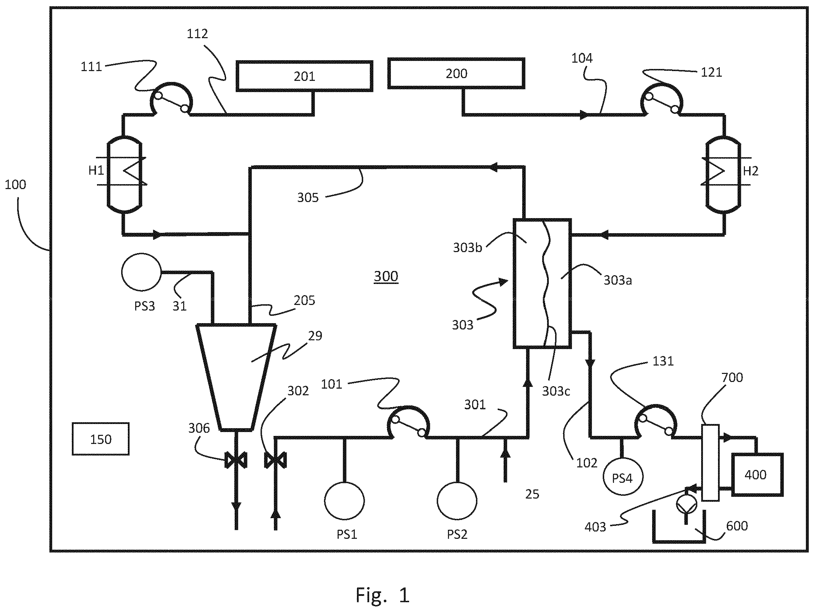

**Fig. 1** zeigt in stark vereinfachter Darstellung eine erfindungsgemäße Blutbehandlungsvorrichtung 100, verbunden mit einem extrakorporalen Blutkreislauf 300.

Der extrakorporale Blutkreislauf 300 weist eine erste Leitung 301, hier in Form eines arteriellen Leitungsabschnitts, auf.

Die erste Leitung 301 steht in Fluidverbindung mit einer Blutbehandlungseinrichtung, hier exemplarisch ein Blutfilter oder Dialysator 303. Der Blutfilter 303 weist eine Dialysierflüssigkeitskammer 303a und eine Blutkammer 303b auf, welche durch eine zumeist semi-permeable Membran 303c voneinander getrennt sind.

Der extrakorporale Blutkreislauf 300 weist ferner wenigstens eine zweite Leitung 305, hier in Form eines venösen Leitungsabschnitts, auf. Sowohl die erste Leitung 301 als auch die zweite Leitung 305 können zu ihrer Verbindung mit dem Gefäßsystem des nicht dargestellten Patienten dienen.

Die erste Leitung 301 ist optional mit einer (ersten) Schlauchklemme 302 zum Sperren oder Schließen der Leitung 301 verbunden. Die zweite Leitung 305 ist optional mit einer (zweiten) Schlauchklemme 306 zum Sperren oder Schließen der Leitung 305 verbunden.

Die in Fig. 1 nur durch einige ihrer Einrichtungen und schematisch repräsentierte Blutbehandlungsvorrichtung 100 weist eine Blutpumpe 101 auf. Die Blutpumpe 101 fördert während der Behandlung des Patienten Blut durch Abschnitte des extrakorporalen Blutkreislaufs 300 und in Richtung zum Blutfilter oder Dialysator 303, wie die kleinen Pfeilspitzen, welche in jeder der Figuren allgemein die Strömungsrichtung angeben, zeigen.

Mittels einer Pumpe für Dialysierflüssigkeit 121, die als Rollenpumpe oder als anderweitig okkludierende Pumpe ausgestaltet sein kann, wird frische Dialysierflüssigkeit aus einer Quelle 200 entlang der Dialysierflüssigkeitszulaufleitung 104 in die Dialysierflüssigkeitskammer 303a gepumpt. Die Dialysierflüssigkeit verlässt die Dialysierflüssigkeitskammer 303a als Dialysat, ggf. angereichert durch Filtrat, in Richtung des Effluentbeutels 400 und wird hierin auch als Effluent bezeichnet.

Die Quelle 200 kann beispielsweise ein Beutel oder ein Container sein. Die Quelle 200 kann ferner eine Fluidleitung sein, aus der online und/oder kontinuierlich erzeugte oder gemischte Flüssigkeit bereitgestellt wird, **z. B.** ein Hydraulikausgang oder -anschluss der Blutbehandlungsvorrichtung 100.

Eine weitere Quelle 201 mit Substituat kann optional vorgesehen sein. Sie kann der Quelle 200 entsprechen oder eine eigene Quelle sein.

Eine nur angedeutete Steuer- oder Regelvorrichtung 150 der Blutbehandlungsvorrichtung 100 kann zur Steuerung und/oder Regelung der Behandlungsvorrichtung vor, während und nach der Behandlung eines Patienten, dazu konfiguriert sein, das erfindungsgemäße Verfahren zu veranlassen oder auszuführen.

Rechts unten ist in Fig. 1 grob angedeutet, dass die erfindungsgemäße Ventilvorrichtung 700 in den Effluentfluss der Effluentzulaufleitung 102 und die Effluentablaufleitung 403 eingreift und den Fluss in den erfindungsgemäßen Effluentbeutel 400 hinein bzw. aus dem erfindungsgemäßen Effluentbeutel 400 heraus ermöglicht bzw. verhindert. Die Wirkweise der Ventilvorrichtung 700 auf die Effluentzulaufleitung 102 und die Effluentablaufleitung 403 sowie ein erfindungsgemäßer Effluentbeutel 400 sind zu den folgenden Figuren näher beschrieben, ebenso seine mögliche, konkrete Ausgestaltung und Anordnung am Effluentbeutel 400 bzw. dessen Auslasshahn 401.

Neben der vorgenannten Blutpumpe 101 weist die in Fig. 1 gezeigte Anordnung ferner rein optional eine Reihe weiterer, jeweils optionaler Pumpen, nämlich die Pumpe 111 für Substituat, die Pumpe 121 für Dialysierflüssigkeit und die Pumpe 131 für das Effluent auf.

Die Pumpe 121 ist vorgesehen, um Dialysierflüssigkeit aus der Quelle 200, beispielsweise einem Beutel, heraus und über eine optional vorhandene Beutelheizung H2 mit einem Beutel dem Blutfilter 303 mittels einer Dialysierflüssigkeitszulaufleitung 104 zuzuführen.

Die auf diese Weise zugeführte Dialysierflüssigkeit tritt über eine Dialysatablaufleitung 102, unterstützt durch die Pumpe 131, wieder aus dem Blutfilter 303 und kann verworfen werden (siehe oben).

Stromauf der Blutpumpe 101 ist ein optionaler arterieller Sensor PS1 vorgesehen. Während einer Behandlung des Patienten misst er den Druck in der arteriellen Leitung.

Stromab der Blutpumpe 101, jedoch stromauf des Blutfilters 303 und, falls vorgesehen, einer Zugabestelle 25 für ein Anticoagulans, beispielsweise Heparin, ist ein weiterer, optionaler Drucksensor PS2 vorgesehen. Er misst den Druck stromauf des Blutfilters 303 ("prä-Hämofilter").

Ein wiederum weiterer Drucksensor kann als PS4 stromab des Blutfilters 303, jedoch vorzugsweise stromauf der Pumpe 131, in der Dialysatablaufleitung 102 zum Messen des Filtratdrucks des Blutfilters 303 vorgesehen sein.

Blut, das den Blutfilter 303 verlässt, durchströmt eine optionale venöse Blutkammer 29, welche eine Entlüftungseinrichtung 31 aufweisen und mit einem weiteren Drucksensor PS3 in Fluidverbindung stehen kann.

In der in Fig. 1 gezeigten exemplarischen Anordnung kann die Steuer- oder Regelvorrichtung 150 mit jeder der hierin genannten Komponenten - jedenfalls oder insbesondere mit der Blutpumpe 101 - in kabelgebundener oder kabelloser Signalverbindung zur Steuerung oder Regelung der Blutbehandlungsvorrichtung 100 stehen. Sie ist optional konfiguriert, um das hierin beschriebene Verfahren auszuführen.

Die optionale Pumpe 111 ist vorgesehen, um Substituat aus der optionalen Quelle 201, beispielsweise einem Beutel, heraus und über eine optional vorhandene Beutelheizung H1 mit einem Beutel der zweiten Leitung 305 zuzuführen.

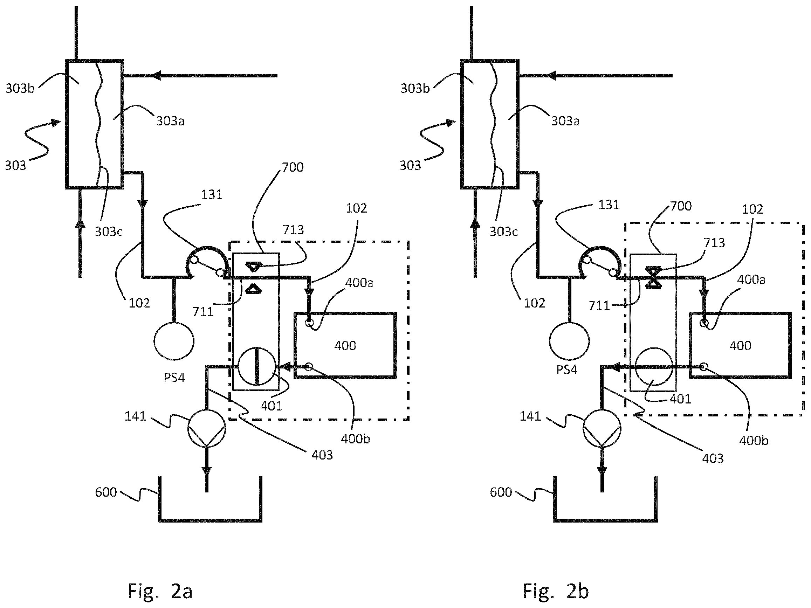

**Fig. 2a** zeigt in vereinfachter Darstellung eine Vielzahl von Leitungen und die Funktionsweise eines erfindungsgemäßen Sets, welches stromab des Blutfilters 303 an einer erfindungsgemäßen Blutbehandlungsvorrichtung 100 angeordnet ist.

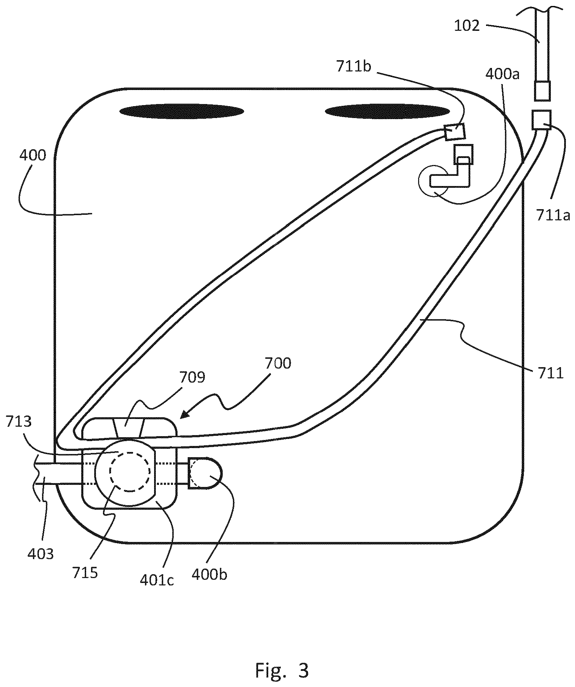

Das Set umfasst eine erfindungsgemäße Ventilvorrichtung 700 sowie eine zweite Fluidleitung 711, welche als Verlängerung der Effluentzulaufleitung 102 verstanden werden kann (siehe Fig. 3).

Weiter umfasst das Set einen Effluentbeutel 400 zum Aufnehmen von Effluent, das bei einer mittels der Blutbehandlungsvorrichtung 100 durchgeführten Blutbehandlung anfällt. Der Effluentbeutel weist seinerseits eine Effluenteinlassöffnung 400a und eine Effluentauslassöffnung 400b auf, wobei die Effluentauslassöffnung 400b einen Auslasshahn 401 oder eine fluidische Verbindung zu einem solchen umfasst.

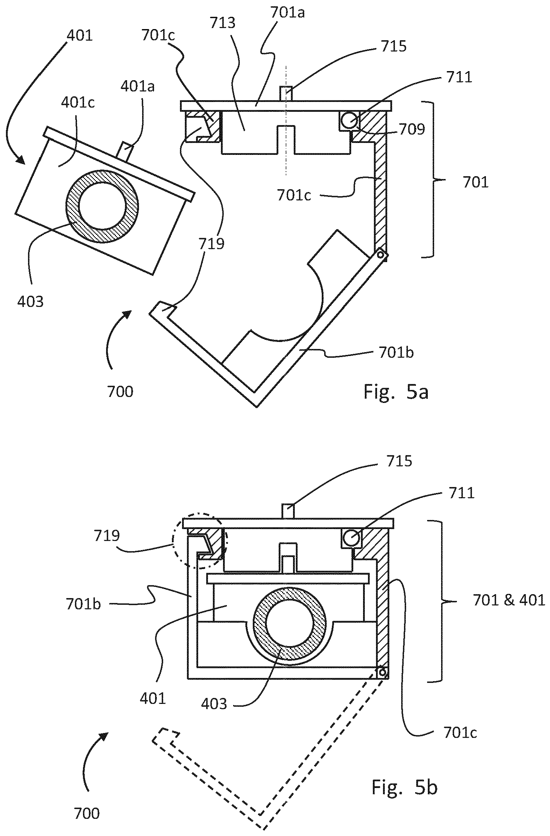

Die Ventilvorrichtung 700 ist konfiguriert, um mittels eines Halteabschnitts 701 (siehe Fig. 5a und Fig. 5b) den Auslasshahn 401 zu halten, vorzugsweise form- und/oder kraftschlüssig.

Ferner zeigt die Anordnung der Fig. 2a eine Effluentpumpe, auch als Filtrat- oder Dialysatpumpe bezeichnet, welche zum Fördern von Effluent ausgehend von einer Dialysierflüssigkeitskammer 303a des Blutfilters 303 in Richtung der Effluenteinlassöffnung 400a des erfindungsgemäßen Effluentbeutels 400 dient. In dem Effluentbeutel 400 wird das Effluent zunächst gesammelt und dann nach der Behandlung bzw. in sogenannten Beutelleerintervallen, z. B. mittels einer optionalen weiteren Pumpe 141 oder mittels Schwerkraft über die Effluentauslassöffnung 400b und über die Effluentablaufleitung 403 in einen optionalen Ausguss 600 verworfen.

Das Beispiel der Fig. 2a zeigt das erfindungsgemäße Set in einem Moment, während welchem dem Effluentbeutel 400 mittels der Effluentpumpe 131 in der Effluentzulaufleitung 102 Effluent aus der Dialysierflüssigkeitskammer 303a des Blutfilters 303 zugeführt wird.

Der Auslasshahn 401, welcher zum Umschalten zwischen zwei Stellungen geeignet ist, ist zwischen der Pumpe 141 und dem Effluentbeutel 400 angeordnet. Er lässt in der gezeigten Stellung keinen Flüssigkeitsstrom durch die Effluentablaufleitung 403 zu. Ein Sperrelement 713 innerhalb der Ventilvorrichtung 700 stromauf des Effluentbeutels 400 befindet sich in einer zweiten Position, in welcher die zweite Fluidleitung 711 geöffnet ist. In der Anordnung des in Fig. 2a gezeigten Moments wird der Effluentbeutel 400, angeordnet an der Blutbehandlungsvorrichtung 100, mit Effluent befüllt, nicht aber entleert, da über die geschlossene Effluentablaufleitung 403 kein Effluent abfließen kann.

Die Pumpe 141, welche in der Effluentablaufleitung 403 angeordnet ist, kann optional wenigstens einen Pumpenantrieb und einen Pumpkopf aufweisen oder hieraus bestehen (nicht in den Figuren gezeigt). Sie ist in der Stellung der Fig. 2a nicht in Betrieb ("OFF"), da die Effluentablaufleitung 403 kein Effluent führt, welches mittels der Pumpe 141 in den Ausguss 600 verworfen werden könnte.

Die Pumpe 141 kann in jeder Ausführungsform eine Rollenpumpe sein. Alternativ ist sie in beliebigen Ausführungsformen keine Rollenpumpe. In manchen Ausführungsformen ist die Pumpe 141 eine Impellerpumpe, in anderen nicht.

**Fig. 2b** zeigt in vereinfachter Darstellung das exemplarische erfindungsgemäße Set aus Fig. 2a.

Alles zum erfindungsgemäßen Set zu Fig. 2a Ausgeführte gilt analog auch in der Fig. 2b.

Die Anordnung der Fig. 2b zeigt das erfindungsgemäße Set in einem Moment, während welchem Effluent mittels der Pumpe 141 über die Effluentablaufleitung 403 aus dem Effluentbeutel 400 herausgefördert und, beispielweise in einen optionalen Ausguss 600, verworfen wird.

Der Auslasshahn 401 lässt in der gezeigten Stellung einen Flüssigkeitsstrom durch die Effluentablaufleitung 403 zu. Ein Sperrelement 713 stromauf des Effluentbeutels 400 befindet sich in einer ersten Position, d. h. die zweite

Fluidleitung 711 ist geschlossen, kann also nicht über das Sperrelement 713 hinweg durchströmt werden.

In der in Fig. 2b gezeigten Stellung bzw. Position wird der (gefüllte) Effluentbeutel 400, angeordnet an der Blutbehandlungsvorrichtung 100, mittels der Pumpe 141 über die nun geöffnete Effluentablaufleitung 403 entleert.

Die Pumpe 141 ist in der Stellung der Fig. 2b in Betrieb ("ON"), da die Effluentablaufleitung 403 nun Effluent führt, welches mittels der Pumpe 141 in den Ausguss 600 verworfen werden kann.

Die vorliegende Erfindung koppelt vorteilhaft die Funktion von Sperrelement 713 und Auslasshahn 401 aneinander. Dies wird in den folgenden Figuren näher beschrieben.

**Fig. 3** zeigt in schematisch vereinfachter Darstellung einen erfindungsgemäßen Effluentbeutel 400 in einer ersten Ausführungsform mit einer erfindungsgemäßen Ventilvorrichtung 700, ebenfalls in einer ersten Ausführungsform.

Der Effluentbeutel 400 weist eine Effluenteinlassöffnung 400a und eine Effluentauslassöffnung 400b auf. Die Effluenteinlassöffnung 400a ist dazu geeignet, mit der Dialysatablaufleitung oder Effluentzulaufleitung 102 mittels geeigneter Verbinder fluidisch verbunden zu werden. Die Effluentauslassöffnung 400b ist dazu geeignet, mit der Effluentablaufleitung 403 mittels geeigneter Verbinder fluidisch verbunden zu werden.

Die Effluentablaufleitung 403 weist einen Auslasshahn 401 auf, welcher geeignet ist einen Flüssigkeitsstrom in der Effluentablaufleitung 403 zu erlauben oder zu unterbinden. Hierzu weist er ein Stellelement 401c (siehe Fig. 5a) auf, welches in eine erste Stellung und in eine zweite Stellung gebracht werden kann. In der ersten Stellung ist der Fluss aus der Effluentauslassöffnung 400b entlang der Effluentablaufleitung 403 gesperrt, in der zweiten Stellung ist Fluss aus der Effluentauslassöffnung 400b entlang der Effluentablaufleitung 403 und somit aus dem Effluentbeutel 400 heraus frei.

Die erfindungsgemäße Ventilvorrichtung 700 kann mittels eines Halteabschnitts 701 am Auslasshahn 401 gehalten oder damit verbunden werden.

In einen Einlegeabschnitt 709 der Ventilvorrichtung 700 kann eine zweite Fluidleitung 711 eingelegt werden. Die zweite Fluidleitung fungiert als "Verlängerung" der Effluentzulaufleitung 102, indem sie mit einem ersten freien Ende 711a mit derselben verbunden werden kann, während das zweite freie Ende 711b der zweiten Fluidleitung 711 mit der Effluenteinlassöffnung 400a oder mit einem daran vorgesehenen Schlauchstück verbunden wird.

Ferner umfasst die Ventilvorrichtung ein Sperrelement 713, welches zwischen einer ersten Position und einer zweiten Position schaltbar ist. Mit dem Sperrelement 713 wird in der ersten Position ein Durchströmen von Flüssigkeit der zweiten Fluidleitung 711 unterbunden, in der zweiten Position wird ein Durchströmen von Flüssigkeit der zweiten Fluidleitung 711 erlaubt.

Stellelement 401c und Sperrelement 713 werden erfindungsgemäß dergestalt gekoppelt oder verbunden, dass das Sperrelement 713 zwangsgeführt in die zweite Position übergeführt wird, wenn das Stellelement 401c in die erste Stellung gebracht wird, und dass das Sperrelement 713 zwangsgeführt in die erste Position übergeführt wird, wenn das Stellelement 401c in die zweite Stellung gebracht wird.

Hieraus ergeben sich zwei Zustände I bzw. II des Abflussschlauchsystems, die folgender Tabelle zu entnehmen sind:

| Zustand | Stellelement 401c | Sperrelement 713 | zweite Fluidleitung 711 | Effluentablaufleitung 403 |
|---|---|---|---|---|
| I | 1. Stellung | 2. Position | offen | geschlossen |
| II | 2. Stellung | 1. Position | geschlossen | offen |

Hierbei ist Zustand I der Zustand, in welchem der Effluentbeutel 400 gefüllt wird (siehe auch Fig. 2a) und Zustand II der Zustand, in welchem der Effluentbeutel 400 geleert wird (siehe auch Fig. 2b).

Das Beispiel der Fig. 3 zeigt den Zustand II der Ventilvorrichtung 700, wobei das Stellelement 401c und die Effluentablaufleitung 403 innerhalb der erfindungsgemäßen Ventilvorrichtung nur mittels gestrichelter Linien angedeutet sind. Außerdem müsste die zweite Fluidleitung 711 mittels ihrer Enden 711a und 711b noch mit der Effluentzulaufleitung 102 bzw. der Effluenteinlassöffnung 400a verbunden werden, um ein funktionsfähiges erfindungsgemäßes Set zu erhalten.

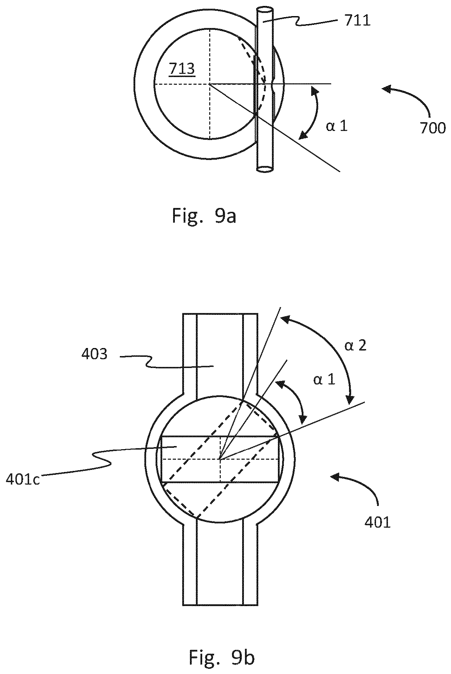

Bei der Betätigung eines Umschaltelements 715 mit oder gegen den Uhrzeigersinn wird die Ventilvorrichtung 700 jeweils vom ersten Zustand I in den zweiten Zustand II geführt, oder umgekehrt. Damit wird sowohl die Stellung des Stellelements 401c als auch die Position des Sperrelements 713 geändert. Vorzugsweise ist das Betätigen des Umschaltelements 715 eine Rotation um 90° um dessen Mittelpunkt, mittels welcher sowohl Sperrelement 713 als auch das Stellelement 401c jeweils zwangsgeführt um den gleichen Winkel mitrotieren. Im Übergang zwischen den beiden Zuständen I und II soll vorzugsweise gewährleistet sein, dass beim Übergang des Stellelements 401c von der ersten Stellung in die zweite Stellung bzw. beim Übergang des Sperrelements 713 von der zweiten Position in die erste Position, und jeweils umgekehrt, in einem bestimmten Winkel, dessen Scheitelpunkt im Mittelpunkt oder auf einer Längsachse des Stellelements bzw. des Sperrelements liegt, vorteilhafterweise in einem bestimmten Winkelbereich, sowohl die zweite Fluidleitung 711 als auch die Effluentablaufleitung 403 unterbrochen sind, d. h. dass durch keine der beiden Leitungen 711, 403 Flüssigkeit fließen kann (siehe Fig. 9a und 9b). Damit können unzulässige Patientenableitströme sicher vermieden werden.

Das Umschaltelement 715 ist in der Fig. 3 lediglich mittels eines gestrichelten Kreises angedeutet.

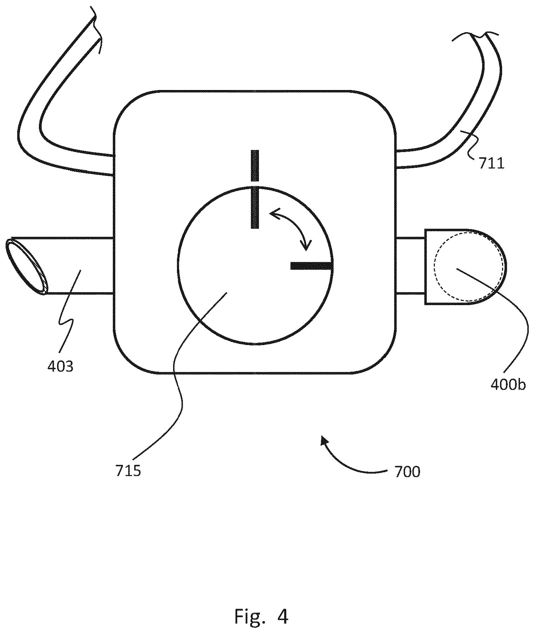

**Fig. 4** zeigt eine exemplarische Draufsicht bzw. eine Außenansicht der erfindungsgemäßen Ventilvorrichtung 700 der Fig. 3 in Vergrößerung.

Zu erkennen ist die geschnitten gezeigte zweite Fluidleitung 711, welche in die Ventilvorrichtung 700 eingelegt ist, und die Effluentablaufleitung 403, welche unten durch die Ventilvorrichtung 700 führt und mit der Effluentauslassöffnung 400b verbunden ist.

Ferner ist das Umschaltelement 715 zu sehen, welches drehbar an der Oberseite der Ventilvorrichtung 700 angeordnet ist. Es dient zum manuellen Umschalten der Ventilvorrichtung 700 aus dem Zustand I in den Zustand II, wie hierin ausgeführt, und umgekehrt.

**Fig. 5a** zeigt in schematisch vereinfachter Darstellung ein erfindungsgemäßes Set in einer beispielhaften Ausführungsform.

Das Set umfasst die erfindungsgemäße Ventilvorrichtung 700, welche ihrerseits einen Halteabschnitt 701 aufweist. Der Halteabschnitt 701 weist seinerseits einen Drehriegel 701a, einen Halter 701c und einen Deckel 701b auf. Der Drehriegel 701a kann in eine Öffnung des Halters 701c eingesetzt sein.

Das Set umfasst ferner eine zweite Fluidleitung 711, welche in einem Einlegeabschnitt 709 der Ventilvorrichtung 700 aufgenommen bzw. in diese eingelegt und durch die Verbindung des Halters 701c mit dem Drehriegel 701a gegen ein Entnehmen gesichert ist. Das Sichern gegen ein Entnehmen kann durch eine Gestaltung des Halters 701c mit Hinterschnitt realisiert sein, in welchen der Drehriegel 701a innen oder außen, vorzugsweise drehbar, radial mit dem Halter 701c verschnappen kann. Der Einlegeabschnitt 709 mit der eingelegten zweiten Fluidleitung 711 ist rechts oben in der Fig. 5a zu erkennen. Die Fluidleitung 711 ist im Querschnitt mit Blick auf die Schnittfläche und in ihr Lumen hinein gezeigt.

Die Fig. 5a zeigt Ventilvorrichtung 700 und Auslasshahn 401 in einem Zustand der Montage, bei welchem die zweite Fluidleitung 711 bereits eingelegt, der Halteabschnitt 701 jedoch noch nicht mit dem Auslasshahn 401 verbunden ist. Der Auslasshahn 401 ist als separates Bauteil links in Fig. 5a zu erkennen.

Deutlich zu sehen ist ein Abschnitt der Effluentablaufleitung 403 im Querschnitt, welche durch den Auslasshahn 401 führt, mit Blick auf die Schnittfläche und in ihr Lumen hinein.

Oben auf dem Auslasshahn 401 befindet sich ein Griffabschnitt 401a, welcher als Verbindungsabschnitt bzw. Kopplungsabschnitt zum Halteabschnitt 701 der erfindungsgemäßen Ventilvorrichtung 700 fungieren kann. Er dient im bestimmungsgemäßen Gebrauch dazu, das Stellelement 401c manuell von seiner ersten Stellung in seine zweite Stellung zu überführen, und umgekehrt.

Der Drehriegel 701a weist oben in der Mitte ein Umschaltelement 715 auf, mittels welchem während der Behandlung die, dann montierte, Ventilvorrichtung 700 manuell zwischen Zustand I und Zustand II, und umgekehrt, geführt werden kann. Zur Beschreibung dieser Zustände sei auf die vorausgehenden Ausführungen verwiesen.

Der Deckel 701b kann aus einer geöffneten Deckelstellung (gezeigt in Fig. 5a) in eine geschlossene Deckelstellung (siehe Fig. 5b) bewegt werden. In der geöffneten Deckelstellung kann der Auslasshahn 401 in den Deckel 701b eingelegt werden. Dabei kann der Griffabschnitt 401a des Auslasshahns 401 in eine Aussparung des Sperrelements 713 eingeschoben oder anderweitig mit dem Sperrelement 713 verbunden werden. Das Sperrelement 713 kann wiederum drehstabil mit dem Drehriegel 701a verbunden oder einstückig hiermit sein.

Deckel 701b und Halter 701c können mittels eines Gelenkabschnitts und/oder eines Schnapp- oder Klicksystems 719 miteinander verbunden oder verbindbar sein. Das Schnapp- oder Klicksystem 719, oder eine alternative Einrichtung, kann den Deckel 701b und den Halter 701c in der zweiten, geschlossenen Deckelstellung halten.

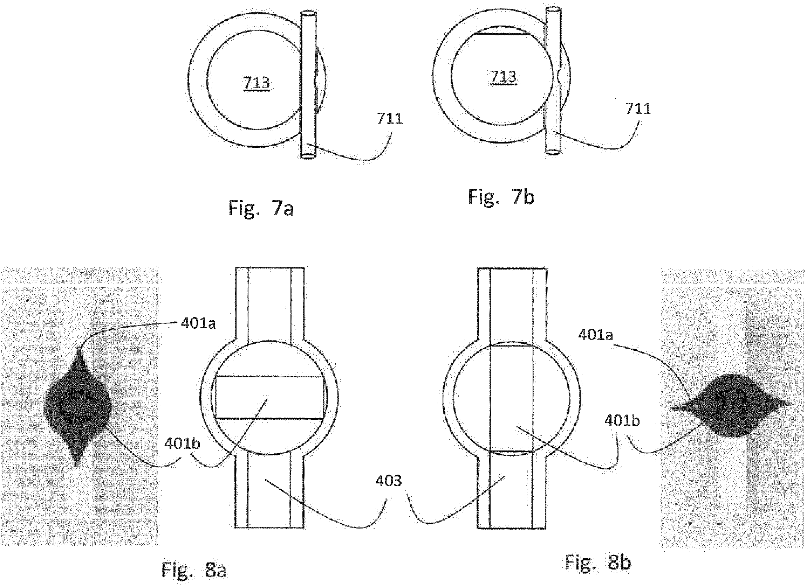

Der Auslasshahn 401 wird zur Verbindung mit der Ventilvorrichtung 700 somit in den Deckel 701b eingelegt. Beim Schließen des Deckels 701b greift ein Steg 401b (Fig. 8a) in das Sperrelement 713 ein. Deckel 701b und Halter 701c umschließen den Auslasshahn 401. Hierzu können der Deckel 701b und/oder der Halter 701c geeignete Aussparungen aufweisen, die beispielsweise die äußere Form des Auslasshahns 401 abbilden können.

**Fig. 5b** zeigt in schematisch vereinfachter Darstellung das erfindungsgemäße Set der Fig. 5a.

Der Deckel 701b ist geschlossen und der Auslasshahn 401 ist in der Ventilvorrichtung 700 aufgenommen bzw. eingelegt. Das Beispiel der Fig. 5b kann auch als montierter Zustand bezeichnet werden.

Die zweite Fluidleitung 711 wird in diesem Beispiel bei Drehen des Drehriegels 701a mittels des Sperrelements 713 durch ihr abschnittsweises, radiales Zusammendrücken vollständig okkludiert.

In der geschlossenen Deckelstellung, die im Beispiel der Fig. 5a und 5b rein exemplarisch mittels einer Nase-NutVerbindung gesichert wird, wird der Auslasshahn 401 sicher im Halter 701c gehalten und mit dem Sperrelement 713 in der oben beschriebenen Art und Weise in Rotationsverbindung gekoppelt. Andere geeignete Vorrichtungen zum Verschließen des Deckels 701b, wie beispielsweise Clip- oder Klammervorrichtungen, sind ebenfalls von der vorliegenden Erfindung umfasst.

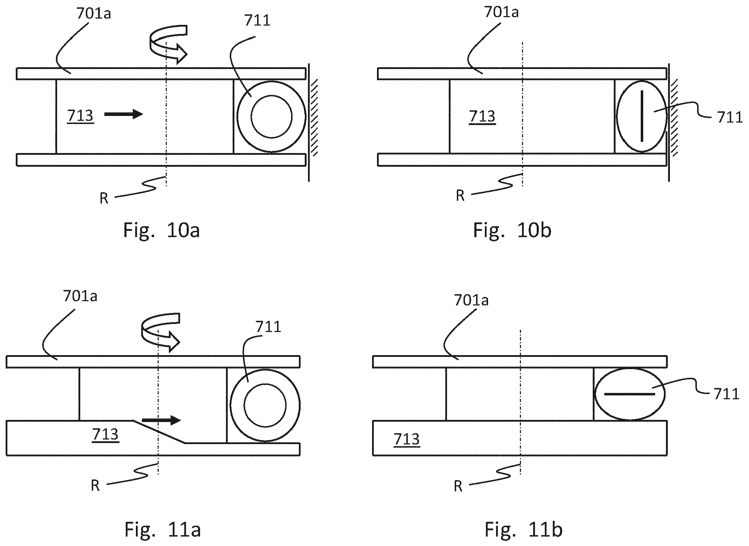

Andere mechanische Lösungen des Sperrelements und/oder Kopplungen desselben mit weiteren Bauteilen, welche die zweite Fluidleitung 711 schließen können, sind ebenfalls von der vorliegenden Erfindung umfasst. Diese können geeignet sein um die zweite Fluidleitung 711 beispielsweise radial oder axial zu verschließen oder vollständig zu okkludieren. Auch translatorische Mechanismen als hierzu geeignete Sperrelemente sind von der vorliegenden Erfindung umfasst. Hierzu wird ferner auf die Beschreibung zu Fig. 10 und 11 verwiesen.

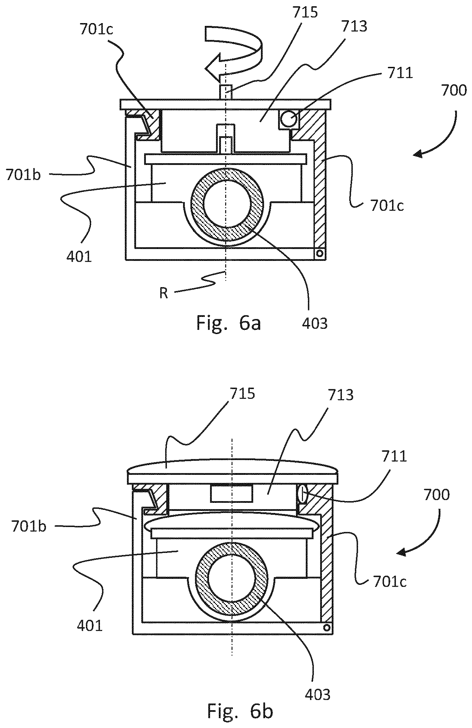

**Fig. 6a** zeigt eine Schnittdarstellung einer erfindungsgemäßen Ventilvorrichtung 700 in einer weiteren Ausführungsform mit einem darin aufgenommenen Auslasshahn 401 in einem (ersten) Zustand I.

Das Lumen der zweiten Fluidleitung 711 ist durchgängig im Bereich der Ventilvorrichtung 700, wohingegen das Lumen der Effluentablaufleitung 403 mittels des Auslasshahns 401 geschlossen ist. Die "Sperrung" der Effluentablaufleitung 403 ist aus dieser Ansicht nicht ersichtlich, da diese nur einen Blick auf die Leitungseingänge des Auslasshahns 401 erlaubt. Die Sperrung wird in den Fig. 8a und 8b näher erläutert.

Der Effluentbeutel 400 (siehe vorausgehende Figuren) würde in diesem Zustand I gefüllt werden können.

Eine Rotationsachse R, um welche Sperrelement 713 und Stellelement 401c mittels Betätigen des vorzugsweise hiermit drehfest vorgesehenen Umschaltelements 715 rotierbar sind, ist in Fig. 6a und den folgenden Figuren durch eine Strich-Punkt-Linie angedeutet.

**Fig. 6b** zeigt eine Schnittdarstellung der Ventilvorrichtung 700 der Fig. 6a mit einem darin aufgenommenen Auslasshahn 401 in einem (zweiten) Zustand II.

Das Umschaltelement 715 wurde um einen Winkel von vorzugsweise 90° gedreht und ist nun von der Seite sichtbar.

Die zweite Fluidleitung ist mittels des Sperrelements 713 geschlossen, während die Effluentablaufleitung 403 mittels Änderung der Stellung des Stellelements 401c geöffnet bzw. ein Durchfluss (wieder) ermöglicht wurde.

Die Sperrung der zweiten Fluidleitung 711 ist in dieser Ansicht gut erkennbar.

Der Effluentbeutel 400 (siehe vorausgehende Figuren) würde in diesem Zustand II entleert werden können.

**Fig. 7a** zeigt eine Ansicht von oben auf ein Sperrelement 713 der erfindungsgemäßen Ventilvorrichtung 700 in einem ersten Zustand I.

Das Sperrelement 713 befindet sich in seiner zweiten Position. Aufgrund der geometrischen Ausgestaltung des Sperrelements 713 gibt dieses in dieser Position eine zweite Fluidleitung 711 frei. Diese kann somit von Effluent durchströmt werden.

**Fig. 7b** zeigt eine Ansicht von oben auf das Sperrelement 713 der Fig. 7a in einem zweiten Zustand II.

Das Sperrelement 713 befindet sich in seiner ersten Position. Aufgrund der geometrischen Ausgestaltung des Sperrelements 713 okkludiert dieses in dieser Position die zweite Fluidleitung 711 vollständig, d. h. eine sich im Gebrauch in der zweiten Fluidleitung 711 befindende, elektrisch leitfähige Flüssigkeitssäule ist elektrisch isolierend unterbrochen. Die zweite Fluidleitung 711 kann somit nicht von Effluent durchströmt werden; Strom kann nicht über eine stehende oder fließende, durchgehende Flüssigkeitssäule aus Effluent über den okkludierten Abschnitt der zweiten Fluidleitung hinweg fließen.

**Fig. 8a** ist im Zusammenhang mit Fig. 7a zu sehen und zeigt eine Ansicht von oben auf ein Stellelement 401c eines Auslasshahns 401, der in den vorgenannten Halter 701c der erfindungsgemäßen Ventilvorrichtung 700 eingelegt werden würde. Links ist der Auslasshahn 401 von außen gezeigt, rechts in einer Längsschnittdarstellung.

Der Auslasshahn 401 befindet sich im ersten Zustand I, d. h. die Effluentablaufleitung 403 ist gesperrt, der Effluentbeutel 400 würde befüllt werden, Effluent könnte nicht aus ihm ablaufen.

In der Außenansicht links ist ein Griffabschnitt 401a des Auslasshahns 401 zu erkennen, welcher als Verbindungsabschnitt zum Drehriegel 701a dienen kann und bei Drehen des Umschaltelements 715 zwangsweise mitrotieren würde. Der Griffabschnitt 401a steht senkrecht zu einem Steg 401b, dessen Stellung von außerhalb des Auslasshahns 401 erkennen lassen kann, ob die Effluentablaufleitung 403 offen oder geschlossen ist. Der Steg kann in manchen Ausführungsformen ebenfalls als Kopplungsabschnitt zwischen Auslasshahn 401 und Ventilvorrichtung 700 fungieren.

Alternativ oder ergänzend kann in manchen Ausführungsformen der Griffabschnitt 401a des Auslasshahns 401 auch als Verbindungsabschnitt zum Sperrelement 713 dienen, um mit diesem zwangsweise mitzurotieren. Dies kann beispielsweise durch Einlegen des Griffabschnitts 401 in eine Nut an der Unterseite des Sperrelements 713 erfolgen.

**Fig. 8b** ist im Zusammenhang mit Fig. 7b zu sehen und zeigt eine Ansicht von oben auf das Stellelement 401c aus Fig. 8a in einer zweiten Stellung. Rechts ist der Auslasshahn 401 von außen gezeigt, links in einer Längsschnittdarstellung.

Der Auslasshahn 401 befindet sich im zweiten Zustand II, d. h. die Effluentablaufleitung 403 ist geöffnet, der Effluentbeutel 400 würde entleert werden, da nun Effluent aus ihm ablaufen kann.

In der Außenansicht links ist zu erkennen, dass der Griffabschnitt 401a nun waagerecht steht, also in einer Position 90° gedreht zu der Position in Fig. 8a. In einer Verbindung mit einem Drehriegel 701a hätte diese Drehung eine Zwangsführung des Sperrabschnitts 713 bewirkt, welche ihrerseits die Sperrung der zweiten Fluidleitung 711 bewirkt hätte (siehe Fig. 7b).

**Fig. 9a** zeigt eine Schnittdarstellung von oben auf ein freigelegt gezeigtes Sperrelement 713 der erfindungsgemäßen Ventilvorrichtung 700 in einem ersten Zustand I der Ventilvorrichtung 700. Das Sperrelement 713 ist in seiner zweiten Position, d. h. durch die zweite Fluidleitung 711 kann Effluent in Richtung Effluenteinlassöffnung 400a fließen.

Während der Rotation des Sperrelements 713 durch Betätigen des Umschaltelements 715 der Ventilvorrichtung 700 gegen den Uhrzeigersinn, um die Ventilvorrichtung 700 in ihren zweiten Zustand II zu überführen, wird die zweite Fluidleitung 711 aufgrund der geometrischen Ausgestaltung des Sperrelements 713 genau ab dem Moment gesperrt, wenn das Sperrelement 713 wenigstens um den Winkel α1 rotiert wurde.

**Fig. 9b** ist im Zusammenhang mit Fig. 9a zu sehen und zeigt eine Schnittdarstellung von oben auf ein Stellelement 401c eines Auslasshahns 401 im ersten Zustand I. Er ist vergleichbar mit dem Zustand I der Fig. 9a. Das Stellelement 401c ist in seiner zweiten Stellung.

Während der Rotation des Stellelements 401c gegen den Uhrzeigersinn, zwangsgeführt durch Koppeln seiner Rotation an eine Betätigung des Umschaltelements 715 der Ventilvorrichtung 700, analog zur Beschreibung zu Fig. 9a, bleibt die Effluentablaufleitung 403 gesperrt, bis um einen Winkel α2 rotiert wurde. Erst bei einer weitergehenden Rotation gegen den Uhrzeigersinn wird die Effluentablaufleitung 403 freigegeben und Effluent kann durch diese hindurch in Richtung Abfluss 600 (siehe Fig. 1 bis 2b) fließen. Somit kann der Effluentbeutel 400 entleert werden.

Wie zu Fig. 3 bereits angedeutet, soll mittels der Ventilvorrichtung 700 vorzugsweise gewährleistet sein, dass während des Übergangs zwischen Zustand I und Zustand II das Stellelement 401c und Sperrelement 713 derart zueinander stehen, dass sowohl die zweite Fluidleitung 711 als auch die Effluentablaufleitung 403 unterbrochen sind, d. h. durch keine der beiden Leitungen 711, 403 kann Flüssigkeit fließen. Dies ist dann der Fall, wenn bautechnisch bewirkt wurde, beispielsweise durch die geometrische Ausgestaltung oder mittels vorteilhafter Dimensionierung, dass die Rotation zum Öffnen der einen Leitung größer sein muss als zum Schließen der anderen. Im Gebrauch ist die Flüssigkeitssäule aufgrund der Okklusion der Leitung 711 im Zustand II unterbrochen, was die elektrische Isolation entlang der - nun unterbrochenen - Flüssigkeitssäule begründet.

Im vorliegenden Beispiel der Fig. 9a und 9b ist dies der Fall, wenn α1 < α2 gilt. Bei einer Stellung des Umschaltelements 715 der Ventilvorrichtung 700 im Winkelbereich zwischen α1 und α2 kann gewährleistet werden, dass durch keine der beiden Leitungen 711 und 403 Flüssigkeit fließen kann. Damit können unzulässige Patientenableitströme sicher vermieden werden.

**Fig. 10a** zeigt einen Querschnitt durch eine erste Ausführungsform eines Sperrelements 713 der erfindungsgemäßen Ventilvorrichtung 700 in dessen zweiter Position, also bei offener zweiter Fluidleitung 711, durch welche in dieser Position Effluent fließen kann.

Das Sperrelement 713 wird bestimmungsgemäß um die Rotationsachse R rotiert werden, angedeutet durch einen geschwungenen Pfeil um die Rotationsachse R, um die Ventilvorrichtung 700 von einem Zustand I in einen Zustand II zu überführen, d. h. die zweite Fluidleitung 711 zu okkludieren.

**Fig. 10b** zeigt das Sperrelement 713 der Fig. 10a in dessen erster Position, also bei vollständig okkludierter zweiter Fluidleitung 711.

Das Sperrelement 713 ist geometrisch derart ausgestaltet, dass mittels einer Rotation desselben ausgehend von seiner zweiten Position in Fig. 10a, vorzugsweise um einen Winkel von ca. 90°, die zweite Fluidleitung 711 durch abschnittsweises Zusammendrücken vollständig okkludiert und damit die elektrisch leitfähige Flüssigkeitssäule in der zweiten Fluidleitung 711 elektrisch isolierend unterbrochen wird. Dies geschieht in radialer Richtung, bezogen auf das Sperrelement 713.

**Fig. 11a** zeigt einen Querschnitt durch eine zweite Ausführungsform eines Sperrelements 713 der erfindungsgemäßen Ventilvorrichtung 700 in dessen zweiter Position, also bei offener zweiter Fluidleitung 711, durch welche in dieser Position Effluent fließen kann.

Analog zur Fig. 10a soll dann das Sperrelement 713 um die Rotationsachse R rotiert werden, angedeutet durch einen geschwungenen Pfeil um die Rotationsachse R, um die Ventilvorrichtung 700 von einem Zustand I in einen Zustand II zu überführen, d. h. die zweite Fluidleitung 711 zu okkludieren.

**Fig. 11b** zeigt das Sperrelement 713 der Fig. 11a in dessen erster Position, also bei vollständig okkludierter zweiter Fluidleitung 711.

Das Sperrelement 713 ist geometrisch, im Beispiel der Fig. 11b in dessen unterem Bereich, derart ausgestaltet, dass mittels einer Rotation desselben ausgehend von seiner zweiten Position in Fig. 11a, vorzugsweise um einen Winkel von ca. 90°, die zweite Fluidleitung 711 durch abschnittsweises Zusammendrücken vollständig okkludiert und damit die elektrisch leitfähige Flüssigkeitssäule in der zweiten Fluidleitung 711 elektrisch isolierend unterbrochen wird. Dies geschieht in axialer Richtung, bezogen auf das Sperrelement 713.

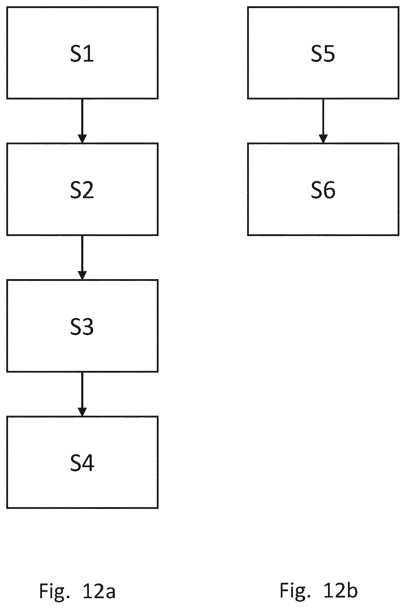

**Fig. 12a** zeigt einen beispielhaften Verlauf eines erfindungsgemäßen Verfahrens zum Vorbereiten eines Effluentbeutels 400 (siehe Fig. 3) zum Aufnehmen von bei einer Blutbehandlung anfallenden Effluents mit folgenden Schritten:
S1 repräsentiert ein Bereitstellen eines erfindungsgemäßen Effluentbeutels 400 oder eines erfindungsgemäßen Sets.
S2 repräsentiert ein Verbinden der zweiten Fluidleitung 711 sowohl mit der Effluentzulaufleitung 102 als auch mit der Effluenteinlassöffnung 400a. Hierzu sind geeignete Verbinder an den freien Enden 711a, 711b der zweiten Fluidleitung 711 vorgesehen.
S3 repräsentiert ein Einlegen eines Schlauchabschnitts der zweiten Fluidleitung 711 in einen Einlegeabschnitt 709 der erfindungsgemäßen Ventilvorrichtung 700, sofern noch nicht im Vorfeld des Verfahrens bzw. bei der Fertigung der Ventilvorrichtung 700 geschehen.
S4 repräsentiert ein Verbinden der Ventilvorrichtung 700 mittels des Halteabschnitts 701 mit dem Auslasshahn 401.

Der Auslasshahn 401 des erfindungsgemäßen Effluentbeutels 400, welcher mittels des erfindungsgemäßen Verfahrens vorbereitet wurde, ist derart mit der erfindungsgemäßen Ventilvorrichtung 700 verbunden, dass bei Betätigen eines Umschaltelements 715 der Ventilvorrichtung 700 sowohl das Sperrelement 713 als auch das Stellelement 401c des Auslasshahns 401 zwangsweise mitgeführt werden (siehe vorausgehende Figuren).

**Fig. 12b** zeigt einen beispielhaften Verlauf eines erfindungsgemäßen Verfahrens zum Entleeren eines erfindungsgemäßen Effluentbeutels 400.

Es umfasst die Schritte:
S5 als ein Bereitstellen eines erfindungsgemäßen Effluentbeutels 400 oder eines erfindungsgemäßen Sets, insbesondere vorbereitet gemäß dem beispielgemäßen Verfahren in Fig. 12a; und
S6 als ein Betätigen des Umschaltelements 715 der Ventilvorrichtung 700 derart, dass eine Fluidverbindung zwischen dem Inneren des Effluentbeutels 400 und dem Inneren der Effluentablaufleitung 403 hergestellt wird.

Die mit dem Auslasshahn 401, gemäß dem zu Fig. 12a ausgeführten Verfahren, verbundene Ventilvorrichtung 700 stellt während einer anstehenden Blutbehandlung vorzugsweise sicher, dass entweder die zweite Fluidleitung 711 oder die Effluentablaufleitung 403 (siehe Fig. 10b) von Effluent durchströmt werden kann. Sie ist mittels des Umschaltelements 715 zwischen den oben ausgeführten Zuständen I und II umschaltbar, d. h. hin- und herschaltbar, so dass immer genau eine oder keine der beiden Leitungen geöffnet ist.

Die Ventilvorrichtung 700 stellt weiter sicher, dass stets entweder die Flüssigkeitssäule der zweiten Fluidleitung 711 aufgrund deren Okklusion im Zustand II oder aber die Flüssigkeitssäule in der Effluentablaufleitung 403 im Zustand I mittels des geschlossenen Auslasshahns 401 jeweils elektrisch isolierend unterbrochen ist, so dass weder im Zustand I noch im Zustand II eine elektrische Erdung des Patienten entlang des Effluents mit dem Ausguss 600 auftreten kann.

Dies bedeutet, dass ein erfindungsgemäßer Effluentbeutel 400 entweder mittels der zweiten Fluidleitung 711 befüllt oder mittels der Effluentablaufleitung 403 entleert werden kann, jedoch nie beides zeitgleich.

### Bezugszeichenliste

- 25: Zugabestelle für Heparin (optional)
- 29: venöse Blutkammer (optional)
- 31: Entlüftungseinrichtung

- 100: Blutbehandlungsvorrichtung
- 101: Blutpumpe
- 102: Dialysatablaufleitung, Effluentzulaufleitung
- 104: Dialysierflüssigkeitszulaufleitung
- 111: Pumpe für Substituat
- 121: Pumpe für Dialysierflüssigkeit
- 131: Pumpe für Dialysat oder Effluent in Effluentzulaufleitung
- 132: Konnektor
- 141: Pumpe in Effluentablaufleitung

- 150: Steuer- oder Regelvorrichtung

- 160: Schlauchklemme in Effluentzulaufleitung

- 200: Quelle mit Dialysierflüssigkeit
- 201: Quelle mit Substituat, optional

- 300: extrakorporaler Blutkreislauf
- 301: erste Leitung (arterieller Leitungsabschnitt)
- 302: (erste) Schlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 306: (zweite) Schlauchklemme
- 400: Effluentbeutel
- 400a: Effluenteinlassöffnung
- 400b: Effluentauslassöffnung

- 401: Auslasshahn
- 401a: Griffabschnitt
- 401b: Steg
- 401c: Stellelement
- 403: Effluentablaufleitung

- 600: Ausguss

- 700: Ventilvorrichtung
- 701: Halteabschnitt
- 701a: Drehriegel
- 701b: Deckel
- 701c: Halter

- 709: Einlegeabschnitt für zweite Fluidleitung
- 711: zweite Fluidleitung
- 711a: erstes freies Ende
- 711b: zweites freies Ende
- 713: Sperrelement
- 715: Umschaltelement
- 717: Umschalthebel
- 719: Schnapp- oder Klicksystem

- H2: Beutelheizung mit Beutel (Dialysierflüssigkeit)
- H1: Beutelheizung mit Beutel (Substituat)

- PS1, PS2: arterieller Drucksensor (optional)
- PS3: Drucksensor (optional)
- PS4: Drucksensor zum Messen des Filtratdrucks

- R: Rotationsachse

- S1, ..., S6: Verfahrensschritte

- α1: erster Winkel
- α2: zweiter Winkel

## Patentansprüche

1. Ventilvorrichtung (700) zum Verbinden mit einem, einer Effluentauslassöffnung (400b) eines
Effluentbeutels (400) zum Aufnehmen von bei einer Blutbehandlung anfallenden Effluents zugeordneten, Auslasshahn (401), welcher ein Stellelement (401c) aufweist und in oder an einer
Effluentablaufleitung (403) oder einem Ansatz hierfür angeordnet ist, wobei das Stellelement (401c) des Auslasshahns (401) in eine erste Stellung, in welcher der Fluss aus der Effluentauslassöffnung (400b) entlang der Effluentablaufleitung (403) gesperrt ist, und in eine zweite Stellung, in welcher der Fluss aus der Effluentauslassöffnung (400b) entlang der Effluentablaufleitung (403) und aus dem
Effluentbeutel (400) heraus freigegeben ist, bringbar ist, wobei der Auslasshahn (401) einen Griffabschnitt aufweist, welcher im bestimmungsgemäßen Gebrauch dazu dient, mit seiner Hilfe das Stellelement (401c) des Auslasshahns (401) manuell von dessen ersten Stellung in dessen zweite Stellung überzuführen, wobei die Ventilvorrichtung (700) aufweist:
- einen Halteabschnitt (701) zum Halten der Ventilvorrichtung (700) an oder auf dem Auslasshahn (401);
- einen Einlegeabschnitt (709) zum Einlegen einer zweiten Fluidleitung (711) in die Ventilvorrichtung (700);
- ein Sperrelement (713), welches zwischen wenigstens einer ersten und einer zweiten Position schaltbar ist;
- ein Umschaltelement (715), angeordnet zum manuellen Umschalten des Sperrelements (713) von der ersten Position in die zweite Position, wobei das Umschaltelement (715) angeordnet ist, dass bei manuellem Umschalten des Sperrelements (713) von der ersten Position in die zweite Position, das Stellelement (401c) oder ein Verbindungsabschnitt zum Verbinden des Umschaltelements (715) mit dem Stellelement (401c) von der zweiten Stellung in die erste Stellung übergeführt wird;
wobei das Sperrelement (713) angeordnet ist, um in der ersten Position direkt oder indirekt auf die zweite Fluidleitung (711) einzuwirken, um einen Fluss entlang der zweiten Fluidleitung (711) zu unterbinden und vorzugsweise im Gebrauch eine elektrisch leitfähige Flüssigkeitssäule in der zweiten Fluidleitung (711) elektrisch isolierend zu unterbrechen, und um in der zweiten Position einen Fluss entlang der zweiten Fluidleitung (711) zuzulassen.

2. Ventilvorrichtung (700) nach Anspruch 1, weiter aufweisend eine Zwangsführung, wobei das Sperrelement (713) derart angeordnet ist, dass, wenn es mittels des Umschaltelements (715) in die zweite Position übergeführt wird, das Stellelement (401c) mittels der Zwangsführung zwangsgeführt in die erste Stellung übergeführt wird, , und derart, dass wenn das das Sperrelement (713) mittels des Umschaltelements (715) in die erste Position übergeführt wird, das Stellelement (401c) zwangsgeführt in die zweite Stellung übergeführt wird.

3. Ventilvorrichtung (700) nach Anspruch 2, wobei das Sperrelement (713) angeordnet ist, um, wenn das Stellelement (401c) in die zweite Stellung übergeführt wird, zwangsgeführt bereits eine Position einzunehmen oder diese beizubehalten, in welcher der Fluss entlang der zweiten Fluidleitung (711) unterbunden ist oder bleibt, und in welcher im Gebrauch vorzugsweise eine elektrisch leitfähige Flüssigkeitssäule in der zweiten Fluidleitung (711) elektrisch isolierend unterbrochen ist, bevor das Stellelement (401c) die zweite Stellung einnimmt.

4. Ventilvorrichtung (700) nach einem der vorangegangenen Ansprüche, wobei der Halteabschnitt (701) einen Drehriegel (701a) mit einem Deckel (701b) aufweist oder hieraus besteht, wobei der Deckel (701b) zwischen einer ersten, geöffneten Deckelstellung zum Einlegen oder Herausnehmen des Auslasshahns (401) aus dem Halteabschnitt (701) oder dem Drehriegel (701a) und einer zweiten Deckelstellung zum kraft- und/oder formschlüssigen Halten des Auslasshahns (401) im Halteabschnitt (701) bewegbar ist.

5. Ventilvorrichtung (700) nach einem der vorangegangenen Ansprüche, wobei der Halteabschnitt (701) einen Drehriegel (701a) zur Aufnahme des Auslasshahns (401) oder eines Abschnitts hiervon und einen Deckel (701b) aufweist, wobei Drehriegel (701a) und/oder Deckel (701b) mit einem Schnapp- oder Klicksystem (719) ausgestaltet sind zu ihrem gemeinsamen oder gegenseitigen Verbinden und/oder zum zumindest teilweisen Verschließen des Drehriegels (701a) mittels des Deckels (701b).

6. Ventilvorrichtung (700) nach einem der vorangegangenen Ansprüche, wobei die zweite Fluidleitung (711) oder ein Abschnitt hiervon in den Einlegeabschnitt (709) eingelegt ist, und/oder wobei der Auslasshahn (401) oder ein Abschnitt hiervon in den Halteabschnitt (701) eingelegt ist, oder wobei der Halteabschnitt (701) mit dem Auslasshahn (401) verbunden ist.

7. Ventilvorrichtung (700) nach Anspruch 6, wobei die zweite Fluidleitung (711) ein erstes freies Ende (711a) zu ihrem Verbinden mit einer Effluentzulaufleitung (102) und ein zweites freies Ende (711b) zu ihrem Verbinden mit einer Effluenteinlassöffnung (400a) eines Effluentbeutels (400) oder einer hiermit verbundenen Fluidleitung aufweist.

8. Ventilvorrichtung (700) nach einem der vorangegangenen Ansprüche, wobei das Sperrelement (713) angeordnet ist, um bei seiner Rotation um einen ersten Winkel (α1) zwischen einer zweiten Position, in welcher die zweite Fluidleitung (711) maximal vom Sperrelement (713) freigegeben ist, und einer ersten Position, in welcher die zweite Fluidleitung (711) maximal durch das Sperrelement (713) geschlossen ist, übergeführt zu werden, wobei das Stellelement (401c) angeordnet ist, um bei seiner Rotation um einen zweiten Winkel (α2) zwischen einer ersten Stellung, in welcher die Effluentablaufleitung (403) maximal vom Stellelement (401c) verschlossen ist, in eine zweite Stellung, in welcher in der Effluentablaufleitung (403) durch das Stellelement (401c) erstmals Fluss erlaubt wird, übergeführt zu werden, wobei der erste Winkel (α1) kleiner ist als der zweite Winkel (α2).

9. Effluentbeutel (400) zum Aufnehmen von bei einer Blutbehandlung anfallenden Effluents, mit
- einer Effluenteinlassöffnung (400a);
- einer Effluentauslassöffnung (400b);
- einem mit der Effluentauslassöffnung (400b) verbundenen Auslasshahn (401), welcher ein Stellelement (401c) aufweist und in oder an einer Effluentablaufleitung (403) angeordnet ist, wobei das Stellelement (401c) in eine erste Stellung des Auslasshahns (401), in welcher der Fluss aus der Effluentauslassöffnung (400b) entlang der Effluentablaufleitung (403) gesperrt ist, und in eine zweite Stellung, in welcher der Fluss aus der Effluentauslassöffnung (400b) entlang der Effluentablaufleitung (403) hindurch und aus dem Effluentbeutel (400) heraus freigegeben ist, bringbar ist; und
- einer Ventilvorrichtung (700) nach einem der vorhergehenden Ansprüche.

10. Set mit einer Ventilvorrichtung (700) nach einem der Ansprüche 1 bis 8 sowie einer zweiten Fluidleitung (711) zum Einlegen in den Einlegeabschnitt (709) der Ventilvorrichtung (700).

11. Set nach Anspruch 10, weiter aufweisend einen Effluentbeutel (400) zum Aufnehmen von bei einer Blutbehandlung anfallenden Effluents, welcher eine Effluenteinlassöffnung (400a) und eine Effluentauslassöffnung (400b) sowie einen Auslasshahn (401) zum Verschließen der Effluentauslassöffnung (400b) oder einem Ansatz hierfür aufweist, wobei die Ventilvorrichtung (700) konfiguriert ist, um mittels ihres Halteabschnitts (701) den Auslasshahn (401) zu halten.

12. Verfahren zum Vorbereiten eines Effluentbeutels (400) zum Aufnehmen von bei einer Blutbehandlung anfallenden Effluents, mit den Schritten:
- Bereitstellen eines Sets nach einem der Ansprüche 10 oder 11 (Schritt S1);
- Verbinden der zweiten Fluidleitung (711) sowohl mit der Effluentzulaufleitung (102) als auch mit der Effluenteinlassöffnung (400a) mittels geeigneter Verbinder an deren freien Enden (711a, 711b) (Schritt S2);
- Einlegen der zweiten Fluidleitung (711) in den Einlegeabschnitt (709) der Ventilvorrichtung (700) sofern noch nicht geschehen (Schritt S3);
- Verbinden der Ventilvorrichtung (700) mittels des Halteabschnitts (701) mit dem Auslasshahn (401) (Schritt S4).

13. Verfahren zum Entleeren eines Effluentbeutels (400), mit den Schritten:
- Bereitstellen eines Sets gemäß einem der Ansprüche 10 bis 11, insbesondere vorbereitet gemäß dem Verfahren des Anspruchs 12 (Schritt S5);
- Betätigen des Umschaltelements (715) der Ventilvorrichtung (700) derart, dass eine Fluidverbindung zwischen dem Inneren des Effluentbeutels (400) und dem Inneren der Effluentablaufleitung (403) hergestellt wird (Schritt S6).

14. Blutbehandlungsvorrichtung (100), verbunden mit einem Effluentbeutel (400) gemäß Anspruch 9 oder mit einem Set gemäß einem der Ansprüche 10 bis 11.

15. Blutbehandlungsvorrichtung nach Anspruch 14, ausgestaltet als Hämodialysevorrichtung, Hämofiltrationsvorrichtung oder Hämodiafiltrationsvorrichtung, insbesondere als eine Vorrichtung für die akute und die chronische Nierenersatztherapie oder für die kontinuierliche Nierenersatztherapie (CRRT = continuous renal replacement therapy).

## Claims

1. A valve device (700) intended to be connected to an outlet tap (401) associated with an effluent outlet opening (400b) of an effluent bag (400) for receiving an effluent produced during a blood treatment, the outlet tap (401) comprising an actuating element (401c) and being arranged in or on an effluent outlet line (403) or at a connection location therefor, wherein the actuating element (401c) of the outlet tap (401) can be brought into a first position, in which the flow is blocked from the effluent outlet opening (400b) along the effluent outlet line (403), and into a second position, in which the flow is allowed from the effluent outlet opening (400b) along the effluent outlet line (403) and out of the effluent bag (400), wherein the outlet tap (401) comprises a handle portion which, during the intended use, serves to manually transfer by means thereof the actuating element (401c) of the outlet tap (401) from its first position to its second position, wherein the valve device (700) comprises:
- a holding portion (701) for holding the valve device (700) on or at the outlet tap (401);
- an insertion portion (709) for inserting a second fluid line (711) into the valve device (700);
- a locking element (713) which can be switched between at least a first and a second position;
- a switching element (715) arranged for manually switching the locking element (713) from the first position to the second position, the switching element (715) being arranged such that, when the locking element (713) is manually switched from the first position to the second position, the actuating element (401c) or a connecting portion for connecting the switching element (715) to the actuating element (401c) is transferred from the second position to the first position;
wherein the locking element (713) is arranged, in the first position, to act directly or indirectly on the second fluid line (711) for preventing a flow along the second fluid line (711), and, preferably for interrupting in an electrically isolating manner a column of electrically conductive liquid in the second fluid line (711) during use, and is arranged, in the second position, to allow a flow along the second fluid line (711).

2. The valve device (700) according to claim 1, further comprising a forced guidance, wherein the locking element (713) is arranged such that, when it is transferred into the second position by means of the switching element (715), the actuating element (401c) is forcibly guided into the first position by means of the forced guidance, and such that, when the locking element (713) is transferred into the first position by means of the switching element (715), the actuating element (401c) is forcibly guided into the second position.

3. The valve device (700) according to claim 2, wherein when the actuating element (401c) is transferred into the second position, the locking element (713) is arranged to assume or maintain an already force-guided position, in which the flow along the second fluid line (711) is or remains prevented, and in which, in use, a column of electrically conductive liquid in the second fluid line (711) is preferably interrupted in an electrically insulating manner before the actuating element (401c) assumes the second position.

4. The valve device (700) according to any one of the preceding claims, wherein the holding portion (701) comprises a rotary latch (701a) with a cap (701b), or consists thereof,
the cap (701b) being movable between a first open cap position for inserting or removing the outlet tap (401) from the holding portion (701) or the rotary latch (701a), and
a second cap position for holding the outlet tap (401) in the holding portion (701) by force and/or form complementarity.

5. The valve device (700) according to any one of the preceding claims, wherein the holding portion (701) comprises a rotary latch (701a) for receiving the outlet tap (401) or a section thereof and a cap (701b), the rotary latch (701a) and/or cap (701b) being designed with a snap or click system (719) for their common or mutual connection and/or for at least partially closing the rotary latch (701a) by means of the cap (701b).

6. The valve device (700) according to any one of the preceding claims, wherein the second fluid line (711) or a portion thereof is inserted into the insertion portion (709), and/or wherein the outlet tap (401) or a portion thereof is inserted into the holding portion (701), or wherein the holding portion (701) is connected to the outlet tap (401).

7. The valve device (700) according to claim 6, wherein the second fluid line (711) comprises a first free end (711a) for connecting it to an effluent inlet line (102) and a second free end (711b) for connecting it to an effluent inlet opening (400a) of an effluent bag (400) or a fluid line connected thereto.

8. The valve device (700) according to any one of the preceding claims, wherein the locking element (713) is arranged to be transferred, upon rotation thereof by a first angle (α1), between a second position in which the second fluid line (711) is maximally allowed by the locking element (713), and a first position in which the second fluid line (711) is maximally closed by the locking element (713), wherein the actuating element (401c) is arranged to be transferred, upon rotation thereof by a second angle (α2), between a first position in which the effluent outlet line (403) is maximally closed by the actuating element (401c), into a second position in which flow is permitted for the first time in the effluent outlet line (403) by the actuating element (401c), wherein the first angle (α1) is smaller than the second angle (α2).

9. An effluent bag (400) for collecting an effluent generated during a blood treatment, comprising:
- an effluent inlet opening (400a);
- an effluent outlet opening (400b);
- an outlet tap (401) connected to the effluent outlet opening (400b), the outlet tap (401) comprising an actuating element (401c) and being arranged in or on an effluent outlet line (403), wherein the actuating element (401c) can be brought into a first position of the outlet tap (401), in which the flow from the effluent outlet opening (400b) along the effluent outlet line (403) is blocked, and into a second position in which the flow from the effluent outlet opening (400b) along the effluent outlet line (403) and out of the effluent bag (400) is permitted; and
- one valve device (700) according to any one of the preceding claims.

10. A set with a valve device (700) according to any one of the claims 1 to 8 as well as a second fluid line (711) for insertion into the insertion portion (709) of the valve device (700).

11. The set according to claim 10, further comprising an effluent bag (400) for collecting an effluent generated during a blood treatment, which comprises an effluent inlet opening (400a) and an effluent outlet opening (400b) as well as an outlet tap (401) for closing the effluent outlet opening (400b) or an attachment therefor, wherein the valve device (700) is configured to hold the outlet tap (401) by means of its holding portion (701).

12. A method for preparing an effluent bag (400) for collecting an effluent generated during a blood treatment, comprising the steps of:
- providing a set according to any one of claims 10 or 11 (step S1);
- connecting the second fluid line (711) to both the effluent inlet line (102) and the effluent inlet opening (400a) by means of suitable connectors at their free ends (711a, 711b) (step S2);
- inserting the second fluid line (711) into the insertion portion (709) of the valve device (700), if not already done (step S3);
- connecting the valve device (700) to the outlet tap (401) by means of the holding portion (701) (step S4).

13. A method for emptying an effluent bag (400), comprising the steps of:
- providing a set according to any one of claims 10 to 11, in particular prepared according to the method of claim 12 (step S5);
- actuating the switching element (715) of the valve device (700) such that a fluid connection is established between the interior of the effluent bag (400) and the interior of the effluent outlet line (403) (step S6).

14. A blood treatment apparatus (100), connected to an effluent bag (400) according to claim 9 or to a set according to any one of the claims 10 to 11.

15. The blood treatment apparatus according to claim 14, designed as a hemodialysis apparatus, hemofiltration apparatus or hemodiafiltration apparatus, in particular as an apparatus for acute and chronic renal replacement therapy or for continuous renal replacement therapy (CRRT).

## Revendications

1. Un dispositif à vanne (700) prévu pour être raccordé à un robinet d'évacuation (401), associé à un orifice d'évacuation d'effluent (400b) d'une poche à effluent (400), destiné à recueillir un effluent généré lors d'un traitement du sang, le robinet d'évacuation (401) comprenant un élément d'actionnement (401c) et étant agencé dans ou sur un conduit d'évacuation d'effluent (403) ou sur un emplacement de raccordement de celui-ci, où l'élément d'actionnement (401c) du robinet d'évacuation (401) peut être amené dans une première position, dans laquelle l'écoulement est bloqué depuis l'orifice d'évacuation d'effluent (400b) le long du conduit d'évacuation d'effluent (403), ainsi que dans une seconde position, dans laquelle l'écoulement est autorisé depuis l'orifice d'évacuation d'effluent (400b) le long du conduit d'évacuation d'effluent (403) et hors de la poche à effluent (400), où le robinet d'évacuation (401) comprend une partie de poignée qui, lors de l'utilisation prévue, sert à déplacer manuellement l'élément d'actionnement (401c) du robinet d'évacuation (401) de sa première position vers sa seconde position à l'aide de celle-ci, où le dispositif à vanne (700) comprend :
- une partie de maintien (701) pour maintenir le dispositif à vanne (700) sur ou au niveau du robinet d'évacuation (401);
- une partie d'insertion (709) pour insérer un second conduit de fluide (711) dans le dispositif à vanne (700);
- un élément de blocage (713) pouvant être commuté entre au moins une première et une seconde position;
- un élément de commutation (715) agencé pour commuter manuellement l'élément de blocage (713) de la première position à la seconde position, l'élément de commutation (715) étant agencé de telle sorte que, lorsque l'élément de blocage (713) est commuté manuellement de la première position à la seconde position, l'élément d'actionnement (401c) ou une partie de raccordement permettant de raccorder l'élément de commutation (715) à l'élément d'actionnement (401c) est transféré(e) de la seconde position à la première position;
où l'élément de blocage (713) est agencé dans la première position pour agir directement ou indirectement sur le second conduit de fluide (711), afin d'empêcher un écoulement le long du second conduit de fluide (711) et, de préférence afin d'interrompre de façon électriquement isolante une colonne de liquide électroconducteur dans le second conduit de fluide (711) lors de l'utilisation, et est agencé dans la seconde position, pour permettre un écoulement le long du second conduit de fluide (711).

2. Le dispositif à vanne (700) selon la première revendication, comportant en outre un guidage forcé, où l'élément de blocage (713) est agencé de telle sorte que, lorsqu'il est transféré dans la seconde position au moyen de l'élément de commutation (715), l'élément d'actionnement (401c) est guidé de force dans la première position au moyen du guidage forcé, et de telle sorte que, lorsque l'élément de blocage (713) est transféré dans la première position au moyen de l'élément de commutation (715), l'élément d'actionnement (401c) est guidé de force dans une seconde position.

3. Le dispositif à vanne (700) selon la revendication 2, où lorsque l'élément d'actionnement (401c) est transféré dans la seconde position, l'élément de blocage (713) est agencé pour prendre ou maintenir une position déjà guidée de force, dans laquelle l'écoulement le long du second conduit de fluide (711) est ou reste empêché, et dans laquelle, lors de l'utilisation, une colonne de liquide électroconducteur dans le second conduit de fluide (711) est de préférence interrompue de façon électriquement isolante avant que l'élément d'actionnement (401c) ne prenne la seconde position.

4. Le dispositif à vanne (700) selon l'une quelconque des revendications précédentes, où la partie de maintien (701) comprend un loquet rotatif (701a) muni d'un capuchon (701b), ou en est constituée, le capuchon (701b) étant mobile entre une première position de capuchon ouverte permettant d'insérer ou de retirer le robinet d'évacuation (401) de la partie de maintien (701) ou du loquet rotatif (701a) et une seconde position de capuchon permettant de maintenir le robinet d'évacuation (401) dans la partie de maintien (701) par complémentarité de force et/ou de forme.

5. Le dispositif à vanne (700) selon l'une quelconque des revendications précédentes, où la partie de maintien (701) comprend un loquet rotatif (701a) destiné à accueillir le robinet d'évacuation (401) ou une partie de celui-ci ainsi qu'un capuchon (701b), le loquet rotatif (701a) et/ou le capuchon (701b) étant conçu(s) avec un système à encliquetage (719) ou à clic pour leur raccordement commun ou mutuel et/ou pour la fermeture au moins partielle du loquet rotatif (701a) au moyen du capuchon (701b).

6. Le dispositif à vanne (700) selon l'une quelconque des revendications précédentes, où le second conduit de fluide (711) ou une partie de celui-ci est inséré (e) dans la partie d'insertion (709), et/ou où le robinet d'évacuation (401) ou une partie de celui-ci est inséré(e) dans la partie de maintien (701), ou où la partie de maintien (701) est raccordée au robinet d'évacuation (401).

7. Le dispositif à vanne (700) selon la revendication 6, où le second conduit de fluide (711) comprend une première extrémité libre (711a) destinée à être raccordée à un conduit d'entrée d'effluent (102) ainsi qu'une seconde extrémité libre (711b) destinée à être raccordée à un orifice d'entrée d'effluent (400a) d'une poche à effluent (400) ou d'un conduit de fluide qui y est raccordé.

8. Le dispositif à vanne (700) selon l'une quelconque des revendications précédentes, où l'élément de blocage (713) est agencé de manière à être transféré, lors de sa rotation d'un premier angle (α1), entre une seconde position dans laquelle le second conduit de fluide (711) est ouvert au maximum par l'élément de blocage (713), et une première position dans laquelle le second conduit de fluide (711) est fermé au maximum par l'élément de blocage (713), où l'élément d'actionnement (401c) est agencé de manière à être transféré, lors de sa rotation d'un second angle (α2), entre une première position dans laquelle le conduit d'évacuation des effluents (403) est fermé au maximum par l'élément d'actionnement (401c), dans une seconde position dans laquelle l'écoulement est autorisé pour la première fois dans le conduit d'évacuation d'effluent (403) par l'élément d'actionnement (401c), où le premier angle (α1) est plus petit que le second angle (α2).

9. Une poche à effluent (400) destinée à recueillir un effluent généré lors d'un traitement du sang, comprenant :
- un orifice d'entrée d'effluent (400a);
- un orifice d'évacuation d'effluent (400b);
- un robinet d'évacuation (401) relié à l'orifice d'évacuation d'effluent (400b), le robinet d'évacuation (401) comprenant un élément d'actionnement (401c) et étant agencé dans ou sur un conduit d'évacuation d'effluent (403), où l'élément d'actionnement (401c) peut être amené dans une première position du robinet d'évacuation (401), dans laquelle l'écoulement depuis l'ouverture d'évacuation d'effluent (400b) le long du conduit d'évacuation d'effluent (403) est bloqué, et dans une seconde position dans laquelle l'écoulement depuis l'orifice d'évacuation d'effluent (400b) le long du conduit d'évacuation d'effluent (403) et hors de la poche à effluent (400) est ouvert; et
- un dispositif à vanne (700) selon l'une quelconque des revendications précédentes.

10. Un ensemble comprenant un dispositif à vanne (700) selon l'une quelconque des revendications 1 à 8 ainsi qu'un second conduit de fluide (711) à insérer dans la partie d'insertion (709) du dispositif à vanne (700).

11. L'ensemble selon la revendication 10, comprenant en outre une poche à effluent (400) destinée à recueillir un effluent généré lors d'un traitement du sang, qui comporte un orifice d'entrée d'effluent (400a) et un orifice d'évacuation d'effluent (400b) ainsi qu'un robinet d'évacuation (401) pour fermer l'orifice d'évacuation d'effluent (400b) ou un accessoire pour celui-ci, le dispositif à vanne (700) étant configuré pour maintenir le robinet d'évacuation (401) au moyen de sa partie de maintien (701).

12. Un procédé de préparation d'une poche à effluent (400) pour recueillir un effluent généré pendant un traitement du sang, comprenant les étapes suivantes :
- fournir un ensemble selon l'une quelconque des revendication 10 ou 11 (étape S1);
- raccorder le second conduit de fluide (711) à la fois au conduit d'entrée d'effluent (102) et à l'orifice d'entrée d'effluent (400a) au moyen de raccords appropriés à leurs extrémités libres (711a, 711b) (étape S2);
- insérer le second conduit de fluide (711) dans la partie d'insertion (709) du dispositif à vanne (700), si ce n'est pas déjà fait (étape S3);
- raccorder le dispositif à vanne (700) au robinet d'évacuation (401) au moyen de la partie de maintien (701) (étape S4).

13. Un procédé pour vider une poche à effluents (400), comprenant les étapes suivantes :
- fournir un ensemble selon l'une quelconque des revendications 10 à 11, notamment préparé selon le procédé de la revendication 12 (étape S5);
- actionner l'élément de commutation (715) du dispositif à vanne (700) de manière à établir une communication fluidique entre l'intérieur de la poche à effluent (400) et l'intérieur du conduit d'évacuation
d'effluent (403) (étape S6).

14. Un appareil de traitement du sang (100), relié à une poche à effluent (400) selon la revendication 9 ou à un ensemble selon l'une quelconque des revendications 10 à 11.

15. L'appareil de traitement du sang selon la revendication 14, conçu comme appareil d'hémodialyse, appareil d'hémofiltration ou appareil d'hémodiafiltration, notamment comme un appareil pour la thérapie de remplacement rénal aigu et chronique ou pour la thérapie de remplacement rénal continu (CRRT = continuous renal replacement therapy).
